# EUROPEAN PATENT APPLICATION

(11) **EP 2 574 680 A1**
(43) Date of publication of application: **03.04.2013**
(21) Application number: 12186078.7
(22) Date of filing: 26.09.2012
(51) Int. Cl.: C12Q 1/68

(54) **Probe, polymorphism detection method, method of evaluating drug efficacy or tolerance, and reagent kit**

(30) Priority: 27.09.2011 JP 2011211373; 04.09.2012 JP 2012194290
(71) Applicant: ARKRAY, Inc., Kyoto-shi, Kyoto 601-8045 (JP)
(72) Inventor: Komori, Mariko, Kyoto-shi Kyoto 602-0008 (JP)
(74) Representative: Jones, Elizabeth Louise

(57) **Abstract**

A probe for detecting a polymorphism in the PIK3 CA gene according to the present invention is at least one fluorescently labeled oligonucleotide selected from P1 to P11' oligonucleotides.

SEQUENCE LISTING

<110>  ARKRAY, Inc.

<120>  Probe, polymorphism detection method, method of evaluating drug
efficacy or tolerance, and reagent kit

<130>  42.13.114278

<150>  JP 2011-211373
<151>  2011-09-27

<150>  JP 2012-194290
<151>  2012-09-04

<160>  38

<170>  PatentIn version 3.4

<210>  1
<211>  420
<212>  DNA
<213>  Artificial Sequence

<220>
<223>  Partial sequence of exon 9 of the PIK3CA gene

<400>  1
tgcaatgaaa ataaattatt ttacaacagt taattagcaa tgtaaaattt attgaaaatg      60

tatttgcttt ttctgtaaat catctgtgaa tccagagggg aaaaatatga caaagaaagc     120

tatataagat attattttat tttacagagt aacagactag ctagagacaa tgaattaagg     180

gaaaatgaca aagaacagct caaagcaatt tctacacgag atcctctctc tgaaatcact     240

gagcaggaga aagattttct atggagtcac aggtaagtgc taaaatggag attctctgtt     300

tctttttctt tattacagaa aaaataactg aatttggctg atctcagcat gtttttacca     360

tacctattgg aataaataaa gcagaattta catgattttt aaactataaa cattgccttt     420


<210>  2
<211>  420
<212>  DNA
<213>  Artificial Sequence

<220>
<223>  Partial sequence of exon 9 of the PIK3CA gene having "G1624A" mutation

<400>  2
tgcaatgaaa ataaattatt ttacaacagt taattagcaa tgtaaaattt attgaaaatg      60

tatttgcttt ttctgtaaat catctgtgaa tccagagggg aaaaatatga caaagaaagc     120

tatataagat attattttat tttacagagt aacagactag ctagagacaa tgaattaagg     180

gaaaatgaca aagaacagct caaagcaatt tctacacgag atcctctctc taaaatcact     240

gagcaggaga aagattttct atggagtcac aggtaagtgc taaaatggag attctctgtt     300

tctttttctt tattacagaa aaaataactg aatttggctg atctcagcat gtttttacca     360

tacctattgg aataaataaa gcagaattta catgattttt aaactataaa cattgccttt     420

<210> 3
<211> 420
<212> DNA
<213> Artificial Sequence

<220>
<223> Partial sequence of exon 9 of the PIK3CA gene having "G1633A" mutation

<400> 3
tgcaatgaaa ataaattatt ttacaacagt taattagcaa tgtaaaattt attgaaaatg 60

tatttgcttt ttctgtaaat catctgtgaa tccagagggg aaaaatatga caaagaaagc 120

tatataagat attattttat tttacagagt aacagactag ctagagacaa tgaattaagg 180

gaaaatgaca aagaacagct caaagcaatt tctacacgag atcctctctc tgaaatcact 240

aagcaggaga aagattttct atggagtcac aggtaagtgc taaaatggag attctctgtt 300

tctttttctt tattacagaa aaaataactg aatttggctg atctcagcat gtttttacca 360

tacctattgg aataaataaa gcagaattta catgattttt aaactataaa cattgccttt 420

<210> 4
<211> 300
<212> DNA
<213> Artificial Sequence

<220>
<223> Partial sequence of exon 20 of the PIK3CA gene

<400> 4
aatctcttca taaatctttt ctcaatgatg cttggctctg gaatgccaga actacaatct 60

tttgatgaca ttgcatacat tcgaaagacc ctagccttag ataaaactga gcaagaggct 120

ttggagtatt tcatgaaaca aatgaatgat gcacatcatg gtggctggac aacaaaaatg 180

gattggatct tccacacaat taaacagcat gcattgaact gaaaagataa ctgagaaaat 240

gaaagctcac tctggattcc acactgcact gttaataact ctcagcaggc aaagaccgat 300

<210> 5
<211> 300
<212> DNA
<213> Artificial Sequence

<220>
<223> Partial sequence of exon 20 of the PIK3CA gene having "A3140G"
mutation

<400> 5
aatctcttca taaatctttt ctcaatgatg cttggctctg gaatgccaga actacaatct 60

tttgatgaca ttgcatacat tcgaaagacc ctagccttag ataaaactga gcaagaggct 120

ttggagtatt tcatgaaaca aatgaatgat gcacgtcatg gtggctggac aacaaaaatg 180

gattggatct tccacacaat taaacagcat gcattgaact gaaaagataa ctgagaaaat 240

gaaagctcac tctggattcc acactgcact gttaataact ctcagcaggc aaagaccgat 300

<210>  6
<211>  19
<212>  DNA
<213>  Artificial Sequence

<220>
<223>  Probe

<400>  6
ctctctgaaa tcactaagc                                                    19


<210>  7
<211>  19
<212>  DNA
<213>  Artificial Sequence

<220>
<223>  Probe

<400>  7
ctctctaaaa tcactgagc                                                    19


<210>  8
<211>  20
<212>  DNA
<213>  Artificial Sequence

<220>
<223>  Probe

<400>  8
caaatgaatg atgcacatca                                                   20


<210>  9
<211>  17
<212>  DNA
<213>  Artificial Sequence

<220>
<223>  Probe

<400>  9
atgcacatca tggtggc                                                      17


<210>  10
<211>  18
<212>  DNA
<213>  Artificial Sequence

<220>
<223>  Probe

<400>  10
atgcacatca tggtggct                                                     18


<210>  11
<211>  17
<212>  DNA

<213>  Artificial Sequence

<220>
<223>  Probe

<400>  11
cagccaccat gacgtgc                                                      17


<210>  12
<211>  18
<212>  DNA
<213>  Artificial Sequence

<220>
<223>  Probe

<400>  12
ccaccatgac gtgcatca                                                     18


<210>  13
<211>  17
<212>  DNA
<213>  Artificial Sequence

<220>
<223>  Probe

<400>  13
caccatgacg tgcatca                                                      17


<210>  14
<211>  17
<212>  DNA
<213>  Artificial Sequence

<220>
<223>  Probe

<400>  14
ccatgacgtg catcatt                                                      17


<210>  15
<211>  17
<212>  DNA
<213>  Artificial Sequence

<220>
<223>  Probe

<400>  15
ccatgacgtg catcatt                                                      17


<210>  16
<211>  19
<212>  DNA
<213>  Artificial Sequence

<220>
<223>  Probe

<400>  16
catgacgtgc atcattcat                                                    19


<210>  17
<211>  19
<212>  DNA
<213>  Artificial Sequence

<220>
<223>  Probe

<400>  17
accatgacgt gcatcattc                                                    19


<210>  18
<211>  17
<212>  DNA
<213>  Artificial Sequence

<220>
<223>  Probe

<400>  18
ccaccatgac gtgcatc                                                      17


<210>  19
<211>  17
<212>  DNA
<213>  Artificial Sequence

<220>
<223>  Probe

<400>  19
cagccaccat gacgtgc                                                      17


<210>  20
<211>  18
<212>  DNA
<213>  Artificial Sequence

<220>
<223>  Probe

<400>  20
ccatgacgtg catcattc                                                     18


<210>  21
<211>  17
<212>  DNA
<213>  Artificial Sequence

<220>
<223>  Probe

<400>  21
ccaccatgac gtgcatc                                                      17


<210>  22

<211>  18
<212>  DNA
<213>  Artificial Sequence

<220>
<223>  Probe

<400>  22
ccatgacgtg catcattc                                                    18


<210>  23
<211>  28
<212>  DNA
<213>  Artificial Sequence

<220>
<223>  Primer

<400>  23
gaacagctca aagcaatttc tacacgag                                         28


<210>  24
<211>  33
<212>  DNA
<213>  Artificial Sequence

<220>
<223>  Primer


<220>
<221>  misc_feature
<222>  (9)..(9)
<223>  y is c or t.

<220>
<221>  misc_feature
<222>  (27)..(27)
<223>  y is c or t.

<400>  24
cagagaatyt ccattttagc acttacytgt gac                                   33


<210>  25
<211>  30
<212>  DNA
<213>  Artificial Sequence

<220>
<223>  Primer

<400>  25
gaggctttgg agtatttcat gaaacaaatg                                       30


<210>  26
<211>  32
<212>  DNA
<213>  Artificial Sequence

<220>
<223>  Primer

<400>  26
gcatgctgtt taattgtgtg gaagatccaa tc                                    32


<210>  27
<211>  16
<212>  DNA
<213>  Artificial Sequence

<220>
<223>  Probe

<400>  27
cacgtcatgg tggctg                                                      16


<210>  28
<211>  17
<212>  DNA
<213>  Artificial Sequence

<220>
<223>  Probe

<400>  28
catcatggtg gctggac                                                     17


<210>  29
<211>  20
<212>  DNA
<213>  Artificial Sequence

<220>
<223>  Probe

<400>  29
catcatggtg gctggacaac                                                  20


<210>  30
<211>  18
<212>  DNA
<213>  Artificial Sequence

<220>
<223>  Probe

<400>  30
acatcatggt ggctggac                                                    18


<210>  31
<211>  19
<212>  DNA
<213>  Artificial Sequence

<220>
<223>  Probe

<400>  31
caaatgaatg atgcacatc                                                   19

<210>  32
<211>  27
<212>  DNA
<213>  Artificial Sequence

<220>
<223>  Probe

<400>  32
cattttgtt gtccagccac catgacg                                          27


<210>  33
<211>  17
<212>  DNA
<213>  Artificial Sequence

<220>
<223>  Probe

<400>  33
ccagccacca tgacgtg                                                    17


<210>  34
<211>  22
<212>  DNA
<213>  Artificial Sequence

<220>
<223>  Probe

<400>  34
acgtgcatca ttcatttgtt tc                                              22


<210>  35
<211>  60
<212>  DNA
<213>  Artificial Sequence

<220>
<223>  Template oligo DNA (wild-type)

<400>  35
tccatttttg ttgtccagcc accatgatgt gcatcattca tttgtttcat gaaatactcc     60


<210>  36
<211>  60
<212>  DNA
<213>  Artificial Sequence

<220>
<223>  Template oligo DNA (mutant-type)

<400>  36
tccatttttg ttgtccagcc accatgacgt gcatcattca tttgtttcat gaaatactcc     60


<210>  37
<211>  60
<212>  DNA
<213>  Artificial Sequence

<220>
<223>  Template oligo DNA (wild-type)

<400>  37
ggagtatttc atgaaacaaa tgaatgatgc acatcatggt ggctggacaa caaaaatgga      60


<210>  38
<211>  60
<212>  DNA
<213>  Artificial Sequence

<220>
<223>  Template oligo DNA (mutant-type)

<400>  38
ggagtatttc atgaaacaaa tgaatgatgc acgtcatggt ggctggacaa caaaaatgga      60

## Description

### BACKGROUND

### Technical Field

The present invention relates to a probe for detecting a polymorphism, a method of detecting a polymorphism, a method of evaluating the efficacy of a drug, and a reagent kit for detecting a polymorphism.

### Related Art

The PIK3CA gene encodes the PI3 kinase which regulates a signaling pathway having an important role in tumorigenesis. In tumor tissues, mutations in the PIK3CA gene are observed in large numbers and amongst them, G1624A(E542K) mutation, G1633A(E545K) mutation and A3140G(H1047R) mutation are observed at a high frequency (see, for example, Science. (2004) Apr 23; 304(5670):554 (Non-patent Document 1) and Oncogene. (2005) Feb 17; 24(8):1477-80 (Non-patent Document 2)).

It has been reported that, in cases where there is a mutation at the above-described three positions, the therapeutic effects of an anti-EGFR antibody drug represented by cetuximab, which is used as a therapeutic agent for unresectable colon cancer, cannot be attained (see, for example, Cancer Res. (2009); 69:(5). March 1, 2009 (Non-patent Document 3) and BMC Cancer. (2011) Mar 25;11:107 (Non-patent Document 4)).

Molecular-targeted drugs such as cetuximab are very expensive and potentially cause serious side-effects such as interstitial pneumonitis. Therefore, it is extremely important to determine the gene mutation relevant to the efficacy of such a drug before administration so as to selectively administer the drug to a patient expected to benefit from the drug efficacy. In this manner, there have been demands for a method by which a gene mutation is measured accurately in a short time as well as inexpensively and easily.

At present, as a method of measuring a gene polymorphism, a method is known in which, after amplifying a region containing a mutation by a PCR method, a nucleic acid probe labeled with a fluorescent dye is hybridized to a target nucleic acid, and the decrease in the amount of light emission by the fluorescent dye is measured so as to analyze a mutation in a base sequence based on the results of melting curve analysis (see Japanese Patent Application Laid-Open (JP-A) No. 2002-119291 (Patent Document 1)).

### SUMMARY OF THE INVENTION

### PROBLEMS TO BE SOLVED BY THE INVENTION

In Non-patent Documents 1 and 3, a mutation in PIK3CA is detected by a direct sequencing method. However, such a direct sequencing method requires labour and cost because, for example, after performing PCR, it is necessary to perform sequence reactions and then electrophoresis using a sequencer. In addition, since it is required to process the resulting amplification product, the amplification product may contaminate the subsequent reaction systems.

In Non-patent Document 2, a mutation in PIK3CA is detected by using a PCR-SSCP (Single Strand Conformation Polymorphism) method and a direct sequencing method in combination. However, such a PCR-SSCP method requires labour and cost because, after performing PCR, the resulting amplification product needs to be subjected to electrophoresis. In addition, since it is required to process the amplification product, the amplification product may contaminate the subsequent reaction systems.

In Non-patent Document 4, a mutation in PIK3CA is detected by the Scorpion ARMS method using a kit manufactured by QIAGEN. However, for the Scorpion ARMS method, it is required to purchase the kit. Further, in addition to the kit, a real-time PCR apparatus is also required, increasing the cost. Moreover, since different reagents and reaction tubes are necessary for each mutation, the labour and the cost are increased.

In Patent Document 1, a polymorphism is detected by a method utilizing a nucleic acid probe. However, with regard to the design of the nucleic acid probe, the disclosure offered in Patent Document 1 is merely that the nucleic acid probe is designed in such a manner that, when a quenching probe whose terminus is labeled with a fluorescent dye is hybridized to its target sequence, at least one G-C pair is formed by the multiple base pairs of the hybrid which is generated between the nucleic acid probe and the target sequence at the terminal. Therefore, in the method described in Patent Document 1, it is required to use a nucleic acid probe having an appropriate sequence for each mutant type.
Furthermore, investigation by the present inventors revealed that the quenching probe does not function sufficiently when the GC content is excessively high in the sequence of the nucleic acid probe.
In view of these present circumstances, a further technical development for detecting a polymorphism in the PIK3CA gene has been strongly desired.

An object of the present invention is to provide a polymorphism detection probe which allows a polymorphism in the PIK3CA gene to be easily detected with high sensitivity and a polymorphism detection method utilizing the probe. Another object of the present invention is to provide a method of evaluating the efficacy of a drug in which the above-described polymorphism detection method is used. Yet another object of the present invention is to provide a reagent kit for detecting a polymorphism which includes the above-described polymorphism detection probe.

### MEANS FOR SOLVING THE PROBLEMS

The present inventors discovered that a polymorphism in the PIK3CA gene can be detected by designing a quenching probe based on a specific region containing a polymorphism in the PIK3CA gene and performing melting curve analysis using the quenching probe. The present invention was achieved based on this discovery.
The present invention is as follows.
<1> A probe for detecting a polymorphism in the PIK3CA gene, which is at least one fluorescently labeled oligonucleotide selected from the following P1 to P11':
(P1) an oligonucleotide having an identity of at least 80% to a base sequence having a length of 7 to 50 bases including the 226th to the 232nd bases of the base sequence indicated in any one of SEQ ID NOs: 1 to 3, wherein the base corresponding to the 226th base is a cytosine labeled with a fluorescent dye;
(P1') an oligonucleotide which hybridizes under stringent conditions to the complementary strand of a base sequence having a length of 7 to 50 bases including the 226th to the 232nd bases of the base sequence indicated in any one of SEQ ID NOs: 1 to 3, wherein the base corresponding to the 226th base is a cytosine labeled with a fluorescent dye;
(P2) an oligonucleotide having an identity of at least 80% to a base sequence having a length of 17 to 50 bases including the 139th to the 155th bases of the base sequence indicated in SEQ ID NO:4 or 5, wherein the base corresponding to the 139th base is a cytosine labeled with a fluorescent dye;
(P2') an oligonucleotide which hybridizes under stringent conditions to the complementary strand of a base sequence having a length of 17 to 50 bases including the 139th to the 155th bases of the base sequence indicated in SEQ ID NO:4 or 5, wherein the base corresponding to the 139th base is a cytosine labeled with a fluorescent dye;
(P3) an oligonucleotide having an identity of at least 80% to a base sequence having a length of 11 to 50 bases including the 155th to the 165th bases of the base sequence indicated in SEQ ID NO:4 or 5, wherein the base corresponding to the 165th base is a cytosine labeled with a fluorescent dye;
(P3') an oligonucleotide which hybridizes under stringent conditions to the complementary strand of a base sequence having a length of 11 to 50 bases including the 155th to the 165th bases of the base sequence indicated in SEQ ID NO:4 or 5, wherein the base corresponding to the 165th base is a cytosine labeled with a fluorescent dye;
(P4) an oligonucleotide having an identity of at least 80% to a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye;
(P4') an oligonucleotide which hybridizes under stringent conditions to the complementary strand of a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye;
(P5) an oligonucleotide having an identity of at least 80% to a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye;
(P5') an oligonucleotide which hybridizes under stringent conditions to the complementary strand of a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye;
(P6) an oligonucleotide having an identity of at least 80% to a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye;
(P6') an oligonucleotide which hybridizes under stringent conditions to the complementary strand of a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye;
(P7) an oligonucleotide having an identity of at least 80% to a base sequence complementary to a base sequence having a length of 7 to 50 bases including the 155th to the 161st bases of the base sequence indicated in SEQ ID NO:4 or 5, wherein the base corresponding to the 161st base is a cytosine labeled with a fluorescent dye;
(P7') an oligonucleotide which hybridizes under stringent conditions to the complementary strand of a base sequence complementary to a base sequence having a length of 7 to 50 bases including the 155th to the 161st bases of the base sequence indicated in SEQ ID NO:4 or 5, wherein the base corresponding to the 161st base is a cytosine labeled with a fluorescent dye;
(P8) an oligonucleotide having an identity of at least 80% to a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye;
(P8') an oligonucleotide which hybridizes under stringent conditions to the complementary strand of a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye;
(P9) an oligonucleotide having an identity of at least 80% to a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye;
(P9') an oligonucleotide which hybridizes under stringent conditions to the complementary strand of a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye;
(P10) an oligonucleotide having an identity of at least 80% to a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye;
(P10') an oligonucleotide which hybridizes under stringent conditions to the complementary strand of a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye;
(P11) an oligonucleotide having an identity of at least 80% to a base sequence complementary to a base sequence having a length of 5 to 50 bases including the 151 st to the 155th bases of the base sequence indicated in SEQ ID NO:4 or 5, wherein the base corresponding to the 151 st base is a cytosine labeled with a fluorescent dye; or
(P11') an oligonucleotide which hybridizes under stringent conditions to the complementary strand of a base sequence complementary to a base sequence having a length of 5 to 50 bases including the 151 st to the 155th bases of the base sequence indicated in SEQ ID NO:4 or 5, wherein the base corresponding to the 151st base is a cytosine labeled with a fluorescent dye.
<2> The probe according to <1>, which is at least one fluorescently labeled oligonucleotide selected from the following P1-1 to P11'-1:
(P1-1) an oligonucleotide having an identity of at least 80% to a base sequence having a length of 7 to 50 bases including the 226th to the 232nd bases of the base sequence indicated in any one of SEQ ID NOs: 1 to 3, wherein the base corresponding to the 226th base is a cytosine labeled with a fluorescent dye, the oligonucleotide recognizing a polymorphism of at least one base selected from the group consisting of the 232nd and the 241 st bases of SEQ ID NO:1;
(P1'-1) an oligonucleotide which hybridizes under stringent conditions to the complementary strand of a base sequence having a length of 7 to 50 bases including the 226th to the 232nd bases of the base sequence indicated in any one of SEQ ID NOs:1 to 3, wherein the base corresponding to the 226th base is a cytosine labeled with a fluorescent dye, the oligonucleotide recognizing a polymorphism of at least one base selected from the group consisting of the 232nd and the 24 1 st bases of SEQ ID NO:1;
(P2-1) an oligonucleotide having an identity of at least 80% to a base sequence having a length of 17 to 50 bases including the 139th to the 155th bases of the base sequence indicated in SEQ ID NO:4 or 5, wherein the base corresponding to the 139th base is a cytosine labeled with a fluorescent dye, the oligonucleotide recognizing a polymorphism at the 155th base of SEQ ID NO:4;
(P2'-1) an oligonucleotide which hybridizes under stringent conditions to the complementary strand of a base sequence having a length of 17 to 50 bases including the 139th to the 155th bases of the base sequence indicated in SEQ ID NO:4 or 5, wherein the base corresponding to the 139th base is a cytosine labeled with a fluorescent dye, the oligonucleotide recognizing a polymorphism at the 155th base of SEQ ID NO:4;
(P3-1) an oligonucleotide having an identity of at least 80% to a base sequence having a length of 11 to 50 bases including the 155th to the 165th bases of the base sequence indicated in SEQ ID NO:4 or 5, wherein the base corresponding to the 165th base is a cytosine labeled with a fluorescent dye, the oligonucleotide recognizing a polymorphism at the 155th base of SEQ ID NO:4;
(P3'-1) an oligonucleotide which hybridizes under stringent conditions to the complementary strand of a base sequence having a length of 11 to 50 bases including the 155th to the 165th bases of the base sequence indicated in SEQ ID NO:4 or 5, wherein the base corresponding to the 165th base is a cytosine labeled with a fluorescent dye, the oligonucleotide recognizing a polymorphism at the 155th base of SEQ ID NO:4;
(P4-1) an oligonucleotide having an identity of at least 80% to a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye, the oligonucleotide recognizing a polymorphism at the 155th base of SEQ ID NO:4;
(P4'-1) an oligonucleotide which hybridizes under stringent conditions to the complementary strand of a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye, the oligonucleotide recognizing a polymorphism at the 155th base of SEQ ID NO:4;
(P5-1) an oligonucleotide having an identity of at least 80% to a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye, the oligonucleotide recognizing a polymorphism at the 155th base of SEQ ID NO:4;
(P5'-1) an oligonucleotide which hybridizes under stringent conditions to the complementary strand of a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye, the oligonucleotide recognizing a polymorphism at the 155th base of SEQ ID NO:4;
(P6-1) an oligonucleotide having an identity of at least 80% to a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye, the oligonucleotide recognizing a polymorphism at the 155th base of SEQ ID NO:4;
(P6'-1) an oligonucleotide which hybridizes under stringent conditions to the complementary strand of a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye, the oligonucleotide recognizing a polymorphism at the 155th base of SEQ ID NO:4;
(P7-1) an oligonucleotide having an identity of at least 80% to a base sequence complementary to a base sequence having a length of 7 to 50 bases including the 155th to the 161st bases of the base sequence indicated in SEQ ID NO:4 or 5, wherein the base corresponding to the 161st base is a cytosine labeled with a fluorescent dye, the oligonucleotide recognizing a polymorphism at the 155th base of SEQ ID NO:4;
(P7'-1) an oligonucleotide which hybridizes under stringent conditions to the complementary strand of a base sequence complementary to a base sequence having a length of 7 to 50 bases including the 155th to the 161st bases of the base sequence indicated in SEQ ID NO:4 or 5, wherein the base corresponding to the 161st base is a cytosine labeled with a fluorescent dye, the oligonucleotide recognizing a polymorphism at the 155th base of SEQ ID NO:4;
(P8-1) an oligonucleotide having an identity of at least 80% to a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye, the oligonucleotide recognizing a polymorphism at the 155th base of SEQ ID NO:4;
(P8'-1) an oligonucleotide which hybridizes under stringent conditions to the complementary strand of a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye, the oligonucleotide recognizing a polymorphism at the 155th base of SEQ ID NO:4;
(P9-1) an oligonucleotide having an identity of at least 80% to a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye, the oligonucleotide recognizing a polymorphism at the 155th base of SEQ ID NO:4;
(P9'-1) an oligonucleotide which hybridizes under stringent conditions to the complementary strand of a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye, the oligonucleotide recognizing a polymorphism at the 155th base of SEQ ID NO:4;
(P10-1) an oligonucleotide having an identity of at least 80% to a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye, the oligonucleotide recognizing a polymorphism at the 155th base of SEQ ID NO:4;
(P10'-1) an oligonucleotide which hybridizes under stringent conditions to the complementary strand of a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye, the oligonucleotide recognizing a polymorphism at the 155th base of SEQ ID NO:4;
(P11-1) an oligonucleotide having an identity of at least 80% to a base sequence complementary to a base sequence having a length of 5 to 50 bases including the 151 st to the 155th bases of the base sequence indicated in SEQ ID NO:4 or 5, wherein the base corresponding to the 151st base is a cytosine labeled with a fluorescent dye, the oligonucleotide recognizing a polymorphism at the 155th base of SEQ ID NO:4; or
(P11'-1) an oligonucleotide which hybridizes under stringent conditions to the complementary strand of a base sequence complementary to a base sequence having a length of 5 to 50 bases including the 151 st to the 155th bases of the base sequence indicated in SEQ ID NO:4 or 5, wherein the base corresponding to the 151st base is a cytosine labeled with a fluorescent dye, the oligonucleotide recognizing a polymorphism at the 155th base of SEQ ID NO:4.
<3> The probe according to <1> or <2>, wherein
the above-described P1 or P1' fluorescently labeled oligonucleotide has the base corresponding to the above-described 226th base labeled with a fluorescent dye at any one of the first to the third positions from the 5'-end;
the above-described P2 or P2' fluorescently labeled oligonucleotide has the base corresponding to the above-described 139th base labeled with a fluorescent dye at any one of the first to the third positions from the 5'-end;
the above-described P3 or P3' fluorescently labeled oligonucleotide has the base corresponding to the above-described 165th base labeled with a fluorescent dye at any one of the first to the third positions from the 3'-end;
the above-described P4 or P4' fluorescently labeled oligonucleotide has the base corresponding to the above-described 167th base labeled with a fluorescent dye at any one of the first to the third positions from the 5'-end;
the above-described P5 or P5' fluorescently labeled oligonucleotide has the base corresponding to the above-described 164th base labeled with a fluorescent dye at any one of the first to the third positions from the 5'-end;
the above-described P6 or P6' fluorescently labeled oligonucleotide has the base corresponding to the above-described 163rd base labeled with a fluorescent dye at any one of the first to the third positions from the 5'-end;
the above-described P7 or P7' fluorescently labeled oligonucleotide has the base corresponding to the above-described 161 st base labeled with a fluorescent dye at any one of the first to the third positions from the 5'-end;
the above-described P8 or P8' fluorescently labeled oligonucleotide has the base corresponding to the above-described 160th base labeled with a fluorescent dye at any one of the first to the third positions from the 5'-end;
the above-described P9 or P9' fluorescently labeled oligonucleotide has the base corresponding to the above-described 144th base labeled with a fluorescent dye at any one of the first to the third positions from the 3'-end;
the above-described P10 or P10' fluorescently labeled oligonucleotide has the base corresponding to the above-described 148th base labeled with a fluorescent dye at any one of the first to the third positions from the 3'-end; or
the above-described P11 or P11' fluorescently labeled oligonucleotide has the base corresponding to the above-described 151 st base labeled with a fluorescent dye at any one of the first to the third positions from the 3'-end.
<4> The probe according to any one of <1> to <3>, wherein
the above-described P1 or P1' fluorescently labeled oligonucleotide has the base corresponding to the above-described 226th base labeled with a fluorescent dye at the 5'-end;
the above-described P2 or P2' fluorescently labeled oligonucleotide has the base corresponding to the above-described 139th base labeled with a fluorescent dye at the 5'-end;
the above-described P3 or P3' fluorescently labeled oligonucleotide has the base corresponding to the above-described 165th base labeled with a fluorescent dye at the 3'-end;
the above-described P4 or P4' fluorescently labeled oligonucleotide has the base corresponding to the above-described 167th base labeled with a fluorescent dye at the 5'-end;
the above-described P5 or P5' fluorescently labeled oligonucleotide has the base corresponding to the above-described 164th base labeled with a fluorescent dye at the 5'-end;
the above-described P6 or P6' fluorescently labeled oligonucleotide has the base corresponding to the above-described 163rd base labeled with a fluorescent dye at the 5'-end;
the above-described P7 or P7' fluorescently labeled oligonucleotide has the base corresponding to the above-described 161st base labeled with a fluorescent dye at the 5'-end;
the above-described P8 or P8' fluorescently labeled oligonucleotide has the base corresponding to the above-described 160th base labeled with a fluorescent dye at the 5'-end;
the above-described P9 or P9' fluorescently labeled oligonucleotide has the base corresponding to the above-described 144th base labeled with a fluorescent dye at the 3'-end;
the above-described P10 or P10' fluorescently labeled oligonucleotide has the base corresponding to the above-described 148th base labeled with a fluorescent dye at the 3'-end; or
the above-described P11 or P11' fluorescently labeled oligonucleotide has the base corresponding to the above-described 151 st base labeled with a fluorescent dye at the 3'-end.
<5> The probe according to any one of <1> to <4>, wherein the above-described fluorescently labeled oligonucleotides emit fluorescence when not hybridized to a target sequence, and the fluorescence intensity of the fluorescently labeled oligonucleotides when hybridized to the target sequence is decreased or increased as compared to when not hybridized to the target sequence.
<6> The probe according to any one of <1> to <5>, which is a probe for melting curve analysis. Alternatively expressed, use of a probe according to any one of <1> to <5> for melting curve analysis.
<7> The probe according to any one of <1> to <6>, which has at least one base sequence selected from the group consisting of SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21 and SEQ ID NO:22.
<8> A method of detecting a polymorphism in the PIK3CA gene, which includes the steps of:
(I) bringing the probe according to any one of <1> to <7> into contact with a single-stranded nucleic acid contained in a sample to hybridize the above-described fluorescently labeled oligonucleotide to the above-described single-stranded nucleic acid, thereby obtaining a hybrid;
(II) dissociating the above-described hybrid by changing the temperature of the sample containing the hybrid to measure the change in fluorescence signal caused by dissociation of the hybrid;
(III) determining the Tm value, which is the dissociation temperature of the hybrid, based on the above-described change in fluorescence signal; and
(IV) based on the above-described Tm value, detecting the presence of a polymorphism of the PIK3CA gene in the above-described single-stranded nucleic acid in the sample.
<9> The method according to <8>, which further includes the step of:
(V) based on the above-described presence of a polymorphism, determining the abundance ratio of the single-stranded nucleic acid having the polymorphism in the single-stranded nucleic acids contained in the above-described sample.
<10> The method according to <8> or <9>, which further includes the step of amplifying the nucleic acid prior to or simultaneously with the above-described step (I) of obtaining a hybrid.
<11> A method of evaluating the efficacy of an anticancer agent, which includes the steps of:
detecting a polymorphism in the PIK3CA gene by the method of detecting a polymorphism according to any one of <8> to <10>; and
determining (or predicting) the efficacy of the anticancer agent based on the detected presence or absence of a polymorphism.
   Said method may be performed on a sample from a subject (e.g. a human) in relation to whom the evaluation is to be made. A sample (as used herein) comprises nucleic acid (e.g. DNA) which may contain said polymorphism.
<12> A reagent kit for detecting a polymorphism in the PIK3CA gene, which includes the probe according to any one of <1> to <7>.
<13> The reagent kit according to <12>, which further includes a primer for amplifying a base sequence containing a region to which the above-described probe hybridizes. The invention also extends to the use of the reagent kit according to <12> or <13> or probe according to <1> to <7> for detecting said polymorphism in the PIK3CA gene.

### EFFECTS OF THE INVENTION

According to the present invention, a polymorphism detection probe which allows a polymorphism in the PIK3CA gene to be easily detected with high sensitivity and a polymorphism detection method utilizing the probe are provided. In addition, the present invention provides a method of evaluating the efficacy of a drug in which the above-described polymorphism detection method is used. Further, the present invention also provides a reagent kit for detecting a polymorphism which includes the above-described polymorphism detection probe.

### BRIEF DESCRIPTION OF THE DRAWINGS

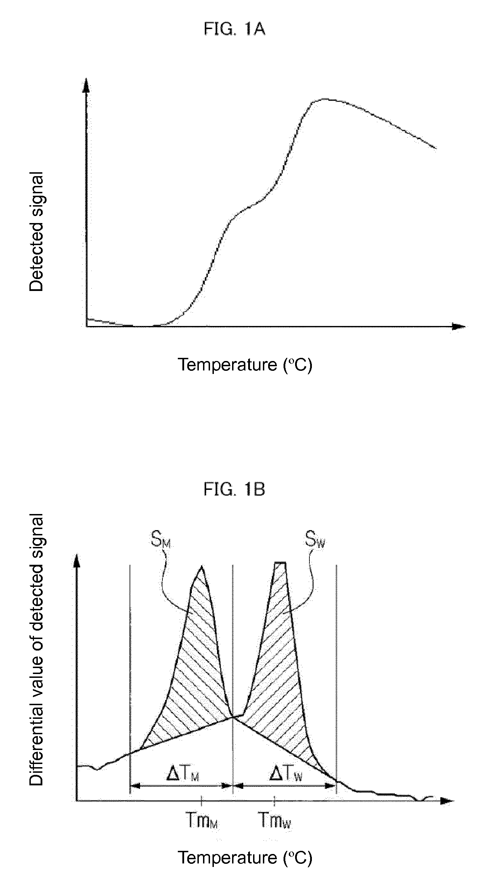

Fig. 1A is an example of a melting curve of a nucleic acid mixture, and Fig. 1B is an example of a differential melting curve of a nucleic acid mixture.
Fig. 2A to Fig 2N are differential melting curves of samples according to Example 1 of the present invention.
Fig. 3A to Fig 3L are differential melting curve of samples according to Example 2 of the present invention.
Fig. 4A to Fig. 4H are differential melting curves of samples according to Comparative Example 1 of the present invention.

### DETAILED DESCRIPTION OF THE INVENTION

The probe for detecting a polymorphism in the PIK3CA gene according to the present invention (hereinafter, may be simply referred to as "the polymorphism detection probe") is a probe for detecting a polymorphism in the PIK3CA gene which is at least one fluorescently labeled oligonucleotide selected from the following P1 to P11':
(P1) an oligonucleotide having an identity of at least 80% to a base sequence having a length of 7 to 50 bases including the 226th to the 232nd bases of the base sequence indicated in any one of SEQ ID NOs: 1 to 3, wherein the base corresponding to the 226th base is a cytosine labeled with a fluorescent dye;
(P1') an oligonucleotide which hybridizes under stringent conditions to the complementary strand of a base sequence having a length of 7 to 50 bases including the 226th to the 232nd bases of the base sequence indicated in any one of SEQ ID NOs: 1 to 3, wherein the base corresponding to the 226th base is a cytosine labeled with a fluorescent dye;
(P2) an oligonucleotide having an identity of at least 80% to a base sequence having a length of 17 to 50 bases including the 139th to the 155th bases of the base sequence indicated in SEQ ID NO:4 or 5, wherein the base corresponding to the 139th base is a cytosine labeled with a fluorescent dye;
(P2') an oligonucleotide which hybridizes under stringent conditions to the complementary strand of a base sequence having a length of 17 to 50 bases including the 139th to the 155th bases of the base sequence indicated in SEQ ID NO:4 or 5, wherein the base corresponding to the 139th base is a cytosine labeled with a fluorescent dye;
(P3) an oligonucleotide having an identity of at least 80% to a base sequence having a length of 11 to 50 bases including the 155th to the 165th bases of the base sequence indicated in SEQ ID NO:4 or 5, wherein the base corresponding to the 165th base is a cytosine labeled with a fluorescent dye;
(P3') an oligonucleotide which hybridizes under stringent conditions to the complementary strand of a base sequence having a length of 11 to 50 bases including the 155th to the 165th bases of the base sequence indicated in SEQ ID NO:4 or 5, wherein the base corresponding to the 165th base is a cytosine labeled with a fluorescent dye;
(P4) an oligonucleotide having an identity of at least 80% to a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye;
(P4') an oligonucleotide which hybridizes under stringent conditions to the complementary strand of a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye;
(P5) an oligonucleotide having an identity of at least 80% to a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye;
(P5') an oligonucleotide which hybridizes under stringent conditions to the complementary strand of a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye;
(P6) an oligonucleotide having an identity of at least 80% to a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye;
(P6') an oligonucleotide which hybridizes under stringent conditions to the complementary strand of a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye;
(P7) an oligonucleotide having an identity of at least 80% to a base sequence complementary to a base sequence having a length of 7 to 50 bases including the 155th to the 161st bases of the base sequence indicated in SEQ ID NO:4 or 5, wherein the base corresponding to the 161st base is a cytosine labeled with a fluorescent dye;
(P7') an oligonucleotide which hybridizes under stringent conditions to the complementary strand of a base sequence complementary to a base sequence having a length of 7 to 50 bases including the 155th to the 161st bases of the base sequence indicated in SEQ ID NO:4 or 5, wherein the base corresponding to the 161st base is a cytosine labeled with a fluorescent dye;
(P8) an oligonucleotide having an identity of at least 80% to a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye;
(P8') an oligonucleotide which hybridizes under stringent conditions to the complementary strand of a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye;
(P9) an oligonucleotide having an identity of at least 80% to a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye;
(P9') an oligonucleotide which hybridizes under stringent conditions to the complementary strand of a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye;
(P10) an oligonucleotide having an identity of at least 80% to a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye;
(P10') an oligonucleotide which hybridizes under stringent conditions to the complementary strand of a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye;
(P11) an oligonucleotide having an identity of at least 80% to a base sequence complementary to a base sequence having a length of 5 to 50 bases including the 151st to the 155th bases of the base sequence indicated in SEQ ID NO:4 or 5, wherein the base corresponding to the 151st base is a cytosine labeled with a fluorescent dye; or
(P11') an oligonucleotide which hybridizes under stringent conditions to the complementary strand of a base sequence complementary to a base sequence having a length of 5 to 50 bases including the 151 st to the 155th bases of the base sequence indicated in SEQ ID NO:4 or 5, wherein the base corresponding to the 151st base is a cytosine labeled with a fluorescent dye.
The method of detecting a polymorphism in the PIK3CA gene according to the present invention is a method which includes detecting a polymorphism in the PIK3CA gene using at least one probe for detecting a polymorphism in the PIK3CA gene as described above.
The method of evaluating the efficacy of a drug according to the present invention is a method which includes detecting a polymorphism in the PIK3CA gene by the above-described method of detecting a polymorphism in the PIK3CA gene, and evaluating (e.g. determining or predicting) the tolerance to a drug or the efficacy of a drug based on the detected presence or absence of the polymorphism.
The reagent kit for detecting a polymorphism according to the present invention is a kit which contains the probe for detecting a polymorphism in the PIK3CA gene.

The "PIK3CA gene" in the present invention is already known, and the base sequence thereof corresponds to the 50001 st to the 91190th bases of NCBI Accession No. NG012113.
The PIK3CA gene encodes the PI3 kinase of the PI3 kinase-Akt signaling pathway which plays an important role in regulating cell differentiation, development, growth and maintenance.

The base sequence of SEQ ID NO:1 is a sequence of the 74541st to the 74960th bases of the NCBI dbSNP Accession No. NG012113 and corresponds to a part of the base sequence of the nucleic acid corresponding to the exon 9 of the PIK3CA gene.
The base sequence of SEQ ID NO:2 has the same base sequence as indicated in SEQ ID NO:1 except that the base corresponding to the 232nd base, G (guanine), is mutated to A (adenine) (hereinafter, also referred to as "G1624A").
The base sequence of SEQ ID NO:3 has the same base sequence as indicated in SEQ ID NO:1 except that the base corresponding to the 24 1 st base, G, is mutated to A (hereinafter, also referred to as "G1633A").

The base sequence of SEQ ID NO:4 is a sequence of the 90621st to the 90920th bases of the NCBI dbSNP Accession No. NG012113 and corresponds to a part of the base sequence of the nucleic acid corresponding to the exon 20 of the PIK3CA gene.
The base sequence of SEQ ID NO:5 has the same base sequence as indicated in SEQ ID NO:4 except that the base corresponding to the 155th base, A, is mutated to G (hereinafter, also referred to as "A3140G").

In the present invention, the description of the base sequences of the sample nucleic acid to be detected in a sample and the polymorphism detection probe or primer shall also apply to complementary base sequences thereof, respectively, unless otherwise specified. Further, when the description of a particular base sequence is applied to a complementary base sequence thereof, the descriptions of base sequences recognized by the particular base sequence in the present invention should be applied provided that the recognition by the particular base sequence is replaced with recognition by a complementary base sequence of the particular base sequence, within a range of the common general technical knowledge of those skilled in the art.

In the present invention, the term "Tm value" is defined as the temperature at which a double-stranded nucleic acid dissociates (dissociation temperature: Tm), and is generally defined as the temperature at which the absorbance at 260 nm has increased by 50% of the total increase in absorbance resulting from complete dissociation of the double-stranded nucleic acid. More specifically, when a solution containing a double-stranded nucleic acid such as a double-stranded DNA is heated, the absorbance at 260 nm of the double-stranded nucleic acid gradually increases. This is because the hydrogen bonds between both strands of the double-stranded DNA are broken by heating, thereby dissociating the double-stranded DNA into single-stranded DNA (melting of DNA). When the double-stranded DNA has completely dissociated into single-stranded DNA, the single-stranded DNA exhibit an absorbance that is about 1.5 times the absorbance at the time of the initiation of the heating (i.e., the absorbance when the entire DNA is in the form of a double-stranded DNA), which serves as an indicator of the completion of the melting. The Tm value is defined based on this phenomenon.
In the present invention, when the phrase "the first to third bases from the 3' end" is used in connection to an oligonucleotide sequence, it is assumed that the base at the 3' end of the oligonucleotide chain is the first base from the 3' end. Similarly, when the phrase "the first to third bases from the 5' end" is used in connection to an oligonucleotide sequence, it is assumed that the base at the 5' end of the oligonucleotide chain is the first base from the 5' end.

In the present specification, the scope of the term "process" or "step" includes not only a discrete process/step, but also a process/step that cannot be clearly distinguished from another process/step as long as the expected effect of the process/step of interest is achieved.
In the present specification, any numerical range expressed using "to" refers to a range including the numerical values before and after "to" as the minimum and maximum values, respectively.
In the case in which the amount of a component that may be included in the composition is indicated in the present invention, when there are multiple substances corresponding to the component in the composition, the indicated amount means the total amount of the multiple substances present in the composition, unless specifically stated otherwise.
In the present specification, the term "mutation" refers to a base sequence newly produced by substitution, deletion, overlapping of or insertion into a part of a wild-type base sequence. Further, the term "polymorphism" refers to a polymorphism of a base sequence caused by a mutation.
The present invention is described below.

<Probe for Detecting Polymorphism in PIK3CA Gene>
The probe for detecting a polymorphism in the PIK3CA gene according to the present invention (hereinafter, may be simply referred to as "the polymorphism detection probe") is a probe for detecting a polymorphism in the PIK3CA gene which is at least one fluorescently labeled oligonucleotide selected from the above-described P1 to P11'.

The above-described P1 to P11' fluorescently labeled oligonucleotides of the present invention also encompass those fluorescently labeled oligonucleotides that are observed to have homology to the sequence defined P1 to P11' fluorescently labeled oligonucleotides.
Specifically, in the present invention, a fluorescently labeled oligonucleotide observed to have homology may be any fluorescently labeled oligonucleotide as long as it exhibits an action comparable to that of the sequence defined P1 to P11' fluorescently labeled oligonucleotides, and such a fluorescently labeled oligonucleotide preferably exhibits an identity of not less than 80% to the sequence defined P1 to P11' oligonucleotides or the corresponding portions of SEQ ID NOs: 1 to 5 or their complementary sequences. From the standpoint of the detection sensitivity, such a fluorescently labeled oligonucleotide may also exhibit an identity of not less than 85%, not less than 90%, not less than 95%, not less than 96%, not less than 97%, not less than 98% or not less than 99%. When the fluorescently labeled oligonucleotide exhibits an identity of not less than 80%, there are advantages in that, for example, a high detection sensitivity is attained for a sample nucleic acid containing a mutant-type PIK3CA gene.

Further, in the present invention, the above-described P1 to P11' fluorescently labeled oligonucleotides also encompass those fluorescently labeled oligonucleotides which hybridize, under stringent conditions, to a complementary strand of the sequence defined P 1 to P11' fluorescently labeled oligonucleotides, as long as they exhibit an action comparable to that of the sequence defined P1 to P11' fluorescently labeled oligonucleotides.

The hybridization may be carried out (under stringent conditions) according to a known method or a method corresponding thereto, such as a method as described in Molecular Cloning 3rd (J. Sambrook et al., Cold Spring Harbor Lab. Press, 2001). This document is incorporated herein by reference.
The term "stringent conditions" means conditions in which specific hybrids are formed, but non-specific hybrids are not formed. Typical examples of the stringent conditions include, for example, conditions in which the hybridization is carried out at a potassium concentration from about 25 mM (e.g. 25 mM) to about 50 mM (e.g. 50 mM) and a magnesium concentration from about 1.0 mM (e.g. 1.0 mM) to about 5.0 mM (e.g. 5.0 mM). One example of the conditions of the present invention is conditions in which the hybridization is carried out in Tris-HCl (pH 8.6), 25 mM KC1, and 1.5 mM MgCl₂, but examples of the conditions of the present invention are not limited thereto. Other examples of the stringent conditions are described in Molecular Cloning 3rd (J. Sambrook et al., Cold Spring Harbor Lab. Press, 2001). This document is incorporated herein by reference.
Those skilled in the art may readily choose such conditions by changing the hybridization reaction and/or the salt concentration of the hybridization reaction solution.

Furthermore, the P1 to P11' fluorescently labeled oligonucleotides of the present invention encompass a fluorescently labeled oligonucleotide having a sequence wherein a base(s) has been inserted to, deleted from and/or substituted in P1 to P11' fluorescently labeled oligonucleotides.
The fluorescently labeled oligonucleotide having a sequence wherein a base(s) has been inserted, deleted and/or substituted is not particularly limited, as long as the oligonucleotide exhibits an effect similar to that of P1 to P11' fluorescently labeled oligonucleotides; and, in cases where a base(s) has been inserted, deleted and/or substituted, the position(s) of the insertion(s), deletion(s) and/or substitution(s) is not particularly limited. The number of bases that has been inserted, deleted and/or substituted may be, for example, 1 base, or 2 or more bases, such as from 1 base to 10 bases and from 1 base to 5 bases, although varying depending on the total length of the fluorescently labeled oligonucleotide.

The oligonucleotides in the above-described fluorescently labeled oligonucleotides also encompass oligonucleotides as well as modified oligonucleotides.
Examples of a structural unit of the above-described oligonucleotide include ribonucleotides, deoxyribonucleotides and artificial nucleic acids. Examples of the artificial nucleic acids include DNAs, RNAs, LNAs (Locked Nucleic Acids) which are RNA analogues, PNAs (Peptide Nucleic Acids) which are peptide nucleic acids, BNAs (Bridged Nucleic Acids) which are cross-linked nucleic acids, and the like.
The above-described oligonucleotides may be constituted by one or multiple types of the structural units described in the above.

In the above-described P1 to P11' fluorescently labeled oligonucleotides, a fluorescently labeled base exists preferably at any one of the first to the third positions from an end of the respective oligonucleotides, and more preferably at an end of the respective oligonucleotides. By this, for example, the sensitivity for detecting a polymorphism is further improved. In addition, the P1 to P11' fluorescently labeled oligonucleotides may be obtained with good productivity.

The details of the fluorescently labeled oligonucleotides according to the present invention will now be described in turn, starting with the P1 and P1' fluorescently labeled oligonucleotides.

The above-described P1 and P1' fluorescently labeled oligonucleotides of the present invention are probes capable of detecting a polymorphism of at least one base selected from the group consisting of the 232nd to the 24 1 st bases of the base sequence indicated in SEQ ID NO:1.

In the wild-type PIK3CA gene, the base corresponding to the 232nd base of the sequence indicated in SEQ ID NO:1 is G (guanine); however, in a mutant-type, the G is mutated to A (adenine) (hereinafter, also referred to as "C1624A"), and this base corresponds to the 1624th base of the PIK3CA gene.

In the wild-type PIK3CA gene, the base corresponding to the 241 st base of the sequence indicated in SEQ ID NO:1 is G (guanine); however, in a mutant-type, the G is mutated to A (adenine) (hereinafter, also referred to as "G1633A"), and this base corresponds to the 1633rd base of the PIK3CA gene.

The above-described P1 fluorescently labeled oligonucleotide of the present invention has an identity of at least 80% to a base sequence having a length of 7 to 50 bases including the 226th to the 232nd bases of the base sequence indicated in any one of SEQ ID NOs: 1 to 3. Here, the base corresponding to the 226th base is a cytosine.

From the standpoint of detection sensitivity, the P1 fluorescently labeled oligonucleotide of the present invention may also exhibit an identity of not less than 85%, not less than 90%, not less than 95%, not less than 96%, not less than 97%, not less than 98% or not less than 99% to a base sequence having a length of 7 to 50 bases including the 226th to the 232nd bases of the base sequence indicated in any one of SEQ ID NOs: 1 to 3. As referred to herein, said 7 to 50 bases are consecutive bases of SEQ ID NOs:1 to 3 which include the 226th to 232nd bases of the base sequence. Similar considerations apply to the other oligonucleotides which are similarly defined. When the identity is less than 80%, the detection sensitivity for a sample nucleic acid containing a mutant-type PIK3CA gene becomes low.

Further, from the standpoint of detecting a polymorphism in the 241st base of the base sequence indicated in SEQ ID NO:1, the form of the P1 fluorescently labeled oligonucleotide also includes a base sequence having an identity of at least 80% to a base sequence having a length of 16 to 50 bases including the 226th to the 24 1 st bases of the base sequence indicated in any one of SEQ ID NOs:1 to 3.
From the standpoint of detection sensitivity, the P1 fluorescently labeled oligonucleotide of the present invention may also exhibit an identity of not less than 85%, not less than 90%, not less than 95%, not less than 96%, not less than 97%, not less than 98% or not less than 99% to a base sequence having a length of 16 to 50 bases including the 226th to the 241st bases of the base sequence indicated in any one of SEQ ID NOs:1 to 3. When the P1 fluorescently labeled oligonucleotide exhibits an identity of not less than 80%, there are advantages in that, for example, a high detection sensitivity is attained for a sample nucleic acid containing a mutant-type PIK3CA gene.

The above-described P1' fluorescently labeled oligonucleotide of the present invention hybridizes, under stringent conditions, to the complementary strand of a base sequence having a length of 7 to 50 bases including the 226th to the 232nd bases of the base sequence indicated in any one of SEQ ID NOs:1 to 3. Here, the base corresponding to the 226th base is a cytosine.
With regard to hybridization and stringent conditions, those matters described in the above are applied. The same applies hereinafter.

Further, from the standpoint of detecting a polymorphism in the 241st base of the base sequence indicated in SEQ ID NO:1, it is more preferred that the P1' fluorescently labeled oligonucleotide take a form in which it hybridizes, under stringent conditions, to the complementary strand of a base sequence having a length of 16 to 50 bases including the 226th to the 241st bases of the base sequence indicated in any one of SEQ ID NOs:1 to 3.

The above-described P1 and P1' fluorescently labeled oligonucleotides of the present invention are required to have a length of 7 mer to 50 mer. When the P1 and P1' fluorescently labeled oligonucleotides have a length shorter than 7 mer or longer than 50 mer, the sensitivity for detecting a polymorphism in the PIK3CA gene is decreased.
Further, the P1 and P1' fluorescently labeled oligonucleotides of the present invention may have a length of 7 mer to 40 mer, 14 mer to 30 mer, or 16 mer to 25 mer. By setting the length in the range of 10 mer to 40 mer, for example, the detection sensitivity tends to be increased.

By changing the base lengths of the P1 and P1' fluorescently labeled oligonucleotides, the Tm value, which is the dissociation temperature of a hybrid formed between the fluorescently labeled oligonucleotides and their respective complementary strands (target sequences), can be adjusted to a desired value.

Table 1 shows examples of the base sequences of the above-described P1 and P1' fluorescently labeled oligonucleotides according to the present invention; however, the present invention is not restricted thereto. Here, in Table 1, the capital letters indicate the bases corresponding to the polymorphisms. The same applies hereinafter.
Further, in Table 1, the underlined cytosine represents the cytosine at the 226th position of the base sequence indicated in any one of SEQ ID NOs:1 to 3.

**[Table 1]**

| | | | SEQ ID NO: |
|---|---|---|---|
| P1 | 5T-PIK3CA G1633A F1-19 | (TAMRA)-ctctctGaaatcactAagc-P | 6 |
| P1 | 5FL-PIK3CA-G1624A-F1 | (BODIPY FL)-ctctctAaaatcactGagc-P | 7 |

Further, in the above-described P1 and P1' fluorescently labeled oligonucleotides of the present invention, it is required that the 226th base (cytosine) is labeled with a fluorescent dye.

The fluorescently labeled oligonucleotide of the present invention may be a fluorescently labeled oligonucleotide in which the fluorescence intensity at the time when the oligonucleotide is hybridized to its target sequence is decreased (quenched) or increased as compared to the fluorescence intensity at the time when the oligonucleotide is not hybridized to its target sequence. In particular, the fluorescently labeled oligonucleotide of the present invention may be a fluorescently labeled oligonucleotide in which the fluorescence intensity at the time when the oligonucleotide is hybridized to its target sequence is decreased as compared to the fluorescence intensity at the time when the oligonucleotide is not hybridized to its target sequence.

A probe that uses the "fluorescence quenching phenomenon" as described above is generally referred to as a guanine quenching probe, and it is known as Q PROBE^{®}. Among such probes, an oligonucleotide which has been designed so that its 3' or 5' end is a cytosine (C) and which has been labeled with a fluorescent dye so that the fluorescence emission is reduced when the C at the 3' or 5' end comes into proximity with a guanine (G) is especially preferable. By using such a probe, the hybridization and dissociation of the probe may be readily checked by the change in its signal.

A known detection method other than the detection method using a Q PROBE^{®} may also be applied. Examples of such a detection method include a TAQ-MAN probe method, a hybridization probe method, a molecular beacon method, and a MGB probe method.

The fluorescent dye is not particularly limited, and examples of the fluorescent dye include fluorescein, phosphor, rhodamine and polymethine dye derivatives. Examples of commercially available products of such fluorescent dyes include Pacific Blue, BODIPY FL, FluorePrime, Fluoredite, FAM, Cy3 and Cy5, and TAMRA.
The detection conditions of the fluorescently-labeled oligonucleotide are not particularly limited, and may be decided, as appropriate, in accordance with the fluorescent dye to be used. For example, Pacific Blue can be detected at a detection wavelength from 445 nm to 480 nm, TAMRA can be detected at a detection wavelength from 585 nm to 700 nm, and BODIPY FL can be detected at a detection wavelength from 520 nm to 555 nm.
By using a probe having such a fluorescent dye, hybridization and dissociation of the probe can be readily confirmed based on the change in fluorescence signal therefrom. Attachment of a fluorescent dye to the oligonucleotide may be carried out according to an ordinary method, such as a method described in JP-A No. 2002-119291.
It should be noted that, in the present invention, the same fluorescent dye may be used, or alternatively, different fluorescent dyes may be used to label one or more of the oligonucleotides.

In addition, the fluorescently-labeled oligonucleotide may have, for example, a phosphate group added to its 3' end. Addition of a phosphate group to the 3' end of the fluorescently-labeled oligonucleotide suppresses elongation of the probe itself by a gene amplification reaction. As described below, DNA in which the presence or absence of a mutation should be detected (target DNA) may be prepared using a gene amplification method such as PCR. When the fluorescently-labeled oligonucleotide that has a phosphate group added to its 3' end is used, the amplification reaction can be carried out even in the presence of the oligonucleotide in the reaction solution of the amplification reaction.
A similar effect can be obtained also by adding a labeling substance (a fluorescent dye) as described above to the 3' end.

The above-described P1 and P1' fluorescently labeled oligonucleotides may be used as a probe for detecting a polymorphism in the PIK3CA gene, particularly at least one of the polymorphisms selected from the group consisting of G1624A and G1633A.
In addition, the probe for detecting a polymorphism in the PIK3CA gene may be used as a probe for melting curve analysis.
The P 1 and P1' fluorescently labeled oligonucleotide according to the present invention may be produced according to a conventional method known as a method for synthesizing an oligonucleotide, such as a method as described in JP-ANo. 2002-119291, except that bases are used so that the base corresponding to the 226th base in the base sequence indicated in SEQ ID NO:1 is a cytosine and the base corresponding to the 226th base is labeled with a fluorescent dye.

More preferable embodiments of the P1 fluorescently labeled oligonucleotide and the P1' fluorescently labeled oligonucleotide include the following P1-1 fluorescently labeled oligonucleotide and the following P1'-1 fluorescently labeled oligonucleotide:
(P1-1) an oligonucleotide having an identity of at least 80% to a base sequence having a length of 7 to 50 bases including the 226th to the 232nd bases of the base sequence indicated in any one of SEQ ID NOs: 1 to 3, wherein the base corresponding to the 226th base is a cytosine labeled with a fluorescent dye, the oligonucleotide recognizing a polymorphism of at least one base selected from the group consisting of the 232nd and the 241 st bases of SEQ ID NO:1; and
(P1'-1) an oligonucleotide which hybridizes under stringent conditions to the complementary strand of a base sequence having a length of 7 to 50 bases including the 226th to the 232nd bases of the base sequence indicated in any one of SEQ ID NOs:1 to 3, wherein the base corresponding to the 226th base is a cytosine labeled with a fluorescent dye, the oligonucleotide recognizing a polymorphism of at least one base selected from the group consisting of the 232nd and the 24 1 st bases of SEQ ID NO:1.
It should be noted that "recognizing a polymorphism of at least one base selected from the group consisting of the 232nd and the 241 st bases of SEQ ID NO:1" has the same meaning as "binding to at least one base selected from the group consisting of the 232nd and the 241st bases of SEQ ID NO:1, which exhibits a polymorphism".

The above-described P2 to P11' fluorescently labeled oligonucleotides will now be described; however, for those matters that are redundant due to the description provided above in relation to the P1 and P1' fluorescently labeled oligonucleotides, the above descriptions shall be referred to.

The above-described P2, P2', P3 and P3' fluorescently labeled oligonucleotides of the present invention are probes capable of detecting a polymorphism at the 155th base of the base sequence indicated in SEQ ID NO:4.

In the wild-type PIK3CA gene, the base corresponding to the 155th base of the sequence indicated in SEQ ID NO:4 is A; however, in a mutant-type, the A is mutated to G (hereinafter, also referred to as "A314OG"), and this base corresponds to the 3140th base of the PIK3 CA gene.

The above-described P2 fluorescently labeled oligonucleotide of the present invention has an identity of at least 80% to a base sequence having a length of 17 to 50 bases including the 139th to the 155th bases of the base sequence indicated in SEQ ID NO:4 or 5. Here, the base corresponding to the 139th base is a cytosine. With regard to the identity, the description provided in relation to the P1 fluorescently labeled oligonucleotide shall be referred to.
The above-described P2' fluorescently labeled oligonucleotide of the present invention hybridizes, under stringent conditions, to the complementary strand of a base sequence having a length of 17 to 50 bases including the 139th to the 155th bases of the base sequence indicated in SEQ ID NO:4 or 5. Here, the base corresponding to the 139th base is a cytosine.

The above-described P3 fluorescently labeled oligonucleotide of the present invention has an identity of at least 80% to a base sequence having a length of 11 to 50 bases including the 155th to the 165th bases of the base sequence indicated in SEQ ID NO:4 or 5. Here, the base corresponding to the 165th base is a cytosine. With regard to the identity, the description provided in relation to the P1 fluorescently labeled oligonucleotide shall be referred to.
The above-described P3' fluorescently labeled oligonucleotide of the present invention hybridizes, under stringent conditions, to the complementary strand of a base sequence having a length of 11 to 50 bases including the 155th to the 165th bases of the base sequence indicated in SEQ ID NO:4 or 5. Here, the base corresponding to the 165th base is a cytosine.

The above-described P2 and P2' fluorescently labeled oligonucleotides of the present invention are required to have a length of 17 mer to 50 mer. When the P2 and P2' fluorescently labeled oligonucleotides have a length shorter than 17 mer or longer than 50 mer, the sensitivity for detecting a polymorphism in the PIK3CA gene is decreased; therefore, such a length is not preferred.
Further, the P2 and P2' fluorescently labeled oligonucleotides of the present invention preferably have a length of 17 mer to 40 mer, more preferably 17 mer to 30 mer, still more preferably 18 mer to 25 mer. By setting the length in the range of 17 mer to 40 mer, for example, the detection sensitivity tends to be increased.

The above-described P3 and P3' fluorescently labeled oligonucleotides of the present invention are required to have a length of 11 mer to 50 mer. When the P3 and P3' fluorescently labeled oligonucleotides have a length shorter than 11 mer or longer than 50 mer, the sensitivity for detecting a polymorphism in the PIK3CA gene is decreased; therefore, such a length is not preferred.
Further, the P3 and P3' fluorescently labeled oligonucleotides of the present invention preferably have a length of 11 mer to 40 mer, more preferably 12 mer to 30 mer, still more preferably 13 mer to 25 mer. By setting the length in the range of 11 mer to 40 mer, for example, the detection sensitivity tends to be increased.

By changing the base lengths of the P2, P2', P3 and P3' fluorescently labeled oligonucleotides, the Tm value, which is the dissociation temperature of a hybrid formed between the fluorescently labeled oligonucleotides and their respective complementary strands (target sequences), can be adjusted to a desired value.

Table 2 shows examples of the base sequences of the above-described P2, P2', P3 and P3' fluorescently labeled oligonucleotides according to the present invention; however, the present invention is not restricted thereto.
Further, in Table 2, the underlined cytosine of SEQ ID NO:8 represents the cytosine at the 139th position of the base sequence indicated in SEQ ID NO:4 or 5. Also, in Table 2, the underlined cytosines of SEQ ID NOs:9 and 10 represent the cytosine at the 165th position of the base sequence indicated in SEQ ID NO:4 or 5.

**[Table 2]**

| | | | SEQ ID NO: |
|---|---|---|---|
| P2 | 5T-PIK3CA3140-Wt-F3 | (TAMRA)-caaatgaatgatgcacAtca-P | 8 |
| P3 | 3T-PIK3CA3140-Wt-F8 | atgcacAtcatggtggc-(TAMRA) | 9 |
| P3 | T-PIK3CA-3140-F10 | atgcacAtcatggtgg(c-(TAMRA))t-P | 10 |

In the above-described P2 and P2' fluorescently labeled oligonucleotides of the present invention, it is required that the 139th base (cytosine) is labeled with a fluorescent dye.
In the above-described P3 and P3' fluorescently labeled oligonucleotides of the present invention, it is required that the 165th base (cytosine) is labeled with a fluorescent dye.

The above-described P2, P2', P3 and P3' fluorescently labeled oligonucleotides may be used as a probe for detecting a polymorphism in the PIK3CA gene, particularly as a probe for detecting a polymorphism in the PIK3CA gene which detects an A3140G mutation.
In addition, the probe for detecting a polymorphism in the PIK3CA gene may be used as a probe for melting curve analysis.

More preferable embodiments of the P2 fluorescently labeled oligonucleotide and the P2' fluorescently labeled oligonucleotide include the following P2-1 fluorescently labeled oligonucleotide and the following P2'-1 fluorescently labeled oligonucleotide:
(P2-1) an oligonucleotide having an identity of at least 80% to a base sequence having a length of 17 to 50 bases including the 139th to the 155th bases of the base sequence indicated in SEQ ID NO:4 or 5, wherein the base corresponding to the 139th base is a cytosine labeled with a fluorescent dye, the oligonucleotide recognizing a polymorphism at the 155th base of SEQ ID NO:4; and
(P2'-1) an oligonucleotide which hybridizes under stringent conditions to the complementary strand of a base sequence having a length of 17 to 50 bases including the 139th to the 155th bases of the base sequence indicated in SEQ ID NO:4 or 5, wherein the base corresponding to the 139th base is a cytosine labeled with a fluorescent dye, the oligonucleotide recognizing a polymorphism at the 155th base of SEQ ID NO:4.

More preferable embodiments of the P3 fluorescently labeled oligonucleotide and the P3' fluorescently labeled oligonucleotide include the following P3-1 fluorescently labeled oligonucleotide and the following P3'-1 fluorescently labeled oligonucleotide:
(P3-1) an oligonucleotide having an identity of at least 80% to a base sequence having a length of 11 to 50 bases including the 155th to the 165th bases of the base sequence indicated in SEQ ID NO:4 or 5, wherein the base corresponding to the 165th base is a cytosine labeled with a fluorescent dye, the oligonucleotide recognizing a polymorphism at the 155th base of SEQ ID NO:4; and
(P3'-1) an oligonucleotide which hybridizes under stringent conditions to the complementary strand of a base sequence having a length of 11 to 50 bases including the 155th to the 165th bases of the base sequence indicated in SEQ ID NO:4 or 5, wherein the base corresponding to the 165th base is a cytosine labeled with a fluorescent dye, the oligonucleotide recognizing a polymorphism at the 155th base of SEQ ID NO:4.

The above-described P4 to P11' fluorescently labeled oligonucleotides of the present invention are probes capable of detecting a polymorphism in a base sequence complementary to the base sequence indicated in SEQ ID NO:4, particularly a polymorphism at the 155th base of the base sequence indicated in SEQ ID NO:4.

The above-described P4 fluorescently labeled oligonucleotide of the present invention has an identity of at least 80% to a base sequence complementary to a base sequence having a length of 13 to 50 bases including the 155th to the 167th bases of the base sequence indicated in SEQ ID NO:4 or 5. Here, the base corresponding to the 167th base is required to be a cytosine. With regard to the identity, those descriptions provided in relation to the P1 fluorescently labeled oligonucleotide shall be referred to. The same applies hereinafter.
The above-described P4' fluorescently labeled oligonucleotide of the present invention hybridizes, under stringent conditions, to the complementary strand of a base sequence complementary to a base sequence having a length of 13 to 50 bases including the 155th to the 167th bases of the base sequence indicated in SEQ ID NO:4 or 5. In other words, the P4' fluorescently labeled oligonucleotide of the present invention hybridizes, under stringent conditions, to a base sequence having a length of 13 to 50 bases including the 155th to the 167th bases of the base sequence indicated in SEQ ID NO:4 or 5. Here, the base corresponding to the 167th base is a cytosine.

The above-described P5 fluorescently labeled oligonucleotide of the present invention has an identity of at least 80% to a base sequence complementary to a base sequence having a length of 10 to 50 bases including the 155th to the 164th bases of the base sequence indicated in SEQ ID NO:4 or 5. Here, the base corresponding to the 164th base is a cytosine.
The above-described P5' fluorescently labeled oligonucleotide of the present invention hybridizes, under stringent conditions, to the complementary strand of a base sequence complementary to a base sequence having a length of 10 to 50 bases including the 155th to the 164th bases of the base sequence indicated in SEQ ID NO:4 or 5. In other words, the P5' fluorescently labeled oligonucleotide of the present invention hybridizes, under stringent conditions, to a base sequence having a length of 10 to 50 bases including the 155th to the 164th bases of the base sequence indicated in SEQ ID NO:4 or 5. Here, the base corresponding to the 164th base is a cytosine.

The above-described P6 fluorescently labeled oligonucleotide of the present invention has an identity of at least 80% to a base sequence complementary to a base sequence having a length of 9 to 50 bases including the 155th to the 163rd bases of the base sequence indicated in SEQ ID NO:4 or 5. Here, the base corresponding to the 163rd base is a cytosine.
The above-described P6' fluorescently labeled oligonucleotide of the present invention hybridizes, under stringent conditions, to the complementary strand of a base sequence complementary to a base sequence having a length of 9 to 50 bases including the 155th to the 163rd bases of the base sequence indicated in SEQ ID NO:4 or 5. In other words, the P6' fluorescently labeled oligonucleotide of the present invention hybridizes, under stringent conditions, to a base sequence having a length of 9 to 50 bases including the 155th to the 163rd bases of the base sequence indicated in SEQ ID NO:4 or 5. Here, the base corresponding to the 163rd base is a cytosine.

The above-described P7 fluorescently labeled oligonucleotide of the present invention has an identity of at least 80% to a base sequence complementary to a base sequence having a length of 7 to 50 bases including the 155th to the 161st bases of the base sequence indicated in SEQ ID NO:4 or 5. Here, the base corresponding to the 161st base is a cytosine.
The above-described P7' fluorescently labeled oligonucleotide of the present invention hybridizes, under stringent conditions, to the complementary strand of a base sequence complementary to a base sequence having a length of 7 to 50 bases including the 155th to the 161st bases of the base sequence indicated in SEQ ID NO:4 or 5. In other words, the P7' fluorescently labeled oligonucleotide of the present invention hybridizes, under stringent conditions, to a base sequence having a length of 7 to 50 bases including the 155th to the 161st bases of the base sequence indicated in SEQ ID NO:4 or 5. Here, the base corresponding to the 161 st base is a cytosine.

The above-described P8 fluorescently labeled oligonucleotide of the present invention has an identity of at least 80% to a base sequence complementary to a base sequence having a length of 6 to 50 bases including the 155th to the 160th bases of the base sequence indicated in SEQ ID NO:4 or 5. Here, the base corresponding to the 160th base is a cytosine.
The above-described P8' fluorescently labeled oligonucleotide of the present invention hybridizes, under stringent conditions, to the complementary strand of a base sequence complementary to a base sequence having a length of 6 to 50 bases including the 155th to the 160th bases of the base sequence indicated in SEQ ID NO:4 or 5. In other words, the P8' fluorescently labeled oligonucleotide of the present invention hybridizes, under stringent conditions, to a base sequence having a length of 6 to 50 bases including the 155th to the 160th bases of the base sequence indicated in SEQ ID NO:4 or 5. Here, the base corresponding to the 160th base is a cytosine.

The above-described P9 fluorescently labeled oligonucleotide of the present invention has an identity of at least 80% to a base sequence complementary to a base sequence having a length of 12 to 50 bases including the 144th to the 155th bases of the base sequence indicated in SEQ ID NO:4 or 5. Here, the base corresponding to the 144th base is a cytosine.
The above-described P9' fluorescently labeled oligonucleotide of the present invention hybridizes, under stringent conditions, to the complementary strand of a base sequence complementary to a base sequence having a length of 12 to 50 bases including the 144th to the 155th bases of the base sequence indicated in SEQ ID NO:4 or 5. In other words, the P9' fluorescently labeled oligonucleotide of the present invention hybridizes, under stringent conditions, to a base sequence having a length of 12 to 50 bases including the 144th to the 155th bases of the base sequence indicated in SEQ ID NO:4 or 5. Here, the base corresponding to the 144th base is a cytosine.

The above-described P10 fluorescently labeled oligonucleotide of the present invention has an identity of at least 80% to a base sequence complementary to a base sequence having a length of 8 to 50 bases including the 148th to the 155th bases of the base sequence indicated in SEQ ID NO:4 or 5. Here, the base corresponding to the 148th base is a cytosine.
The above-described P10' fluorescently labeled oligonucleotide of the present invention hybridizes, under stringent conditions, to the complementary strand of a base sequence complementary to a base sequence having a length of 8 to 50 bases including the 148th to the 155th bases of the base sequence indicated in SEQ ID NO:4 or 5. In other words, the P10' fluorescently labeled oligonucleotide of the present invention hybridizes, under stringent conditions, to a base sequence having a length of 8 to 50 bases including the 148th to the 155th bases of the base sequence indicated in SEQ ID NO:4 or 5. Here, the base corresponding to the 148th base is a cytosine.

The above-described P11 fluorescently labeled oligonucleotide of the present invention has an identity of at least 80% to a base sequence complementary to a base sequence having a length of 5 to 50 bases including the 151 st to the 155th bases of the base sequence indicated in SEQ ID NO:4 or 5. Here, the base corresponding to the 151st base is a cytosine.
The above-described P11' fluorescently labeled oligonucleotide of the present invention hybridizes, under stringent conditions, to the complementary strand of a base sequence complementary to a base sequence having a length of 5 to 50 bases including the 151st to the 155th bases of the base sequence indicated in SEQ ID NO:4 or 5. In other words, the P11' fluorescently labeled oligonucleotide of the present invention hybridizes, under stringent conditions, to a base sequence having a length of 5 to 50 bases including the 151st to the 155th bases of the base sequence indicated in SEQ ID NO:4 or 5. Here, the base corresponding to the 151 st base is a cytosine.

The above-described P4 and P4' fluorescently labeled oligonucleotides of the present invention are required to have a length of 13 mer to 50 mer. When the P4 and P4' fluorescently labeled oligonucleotides have a length shorter than 13 mer or longer than 50 mer, the sensitivity for detecting a polymorphism in the PIK3CA gene is decreased; therefore, such a length is not preferred.
Further, the P4 and P4' fluorescently labeled oligonucleotides of the present invention preferably have a length of 13 mer to 40 mer, more preferably 13 mer to 30 mer, still more preferably 14 mer to 25 mer. By setting the length in the range of 13 mer to 40 mer, for example, the detection sensitivity tends to be increased.

The above-described P5 and P5' fluorescently labeled oligonucleotides of the present invention are required to have a length of 10 mer to 50 mer. When the P5 and P5' fluorescently labeled oligonucleotides have a length shorter than 10 mer or longer than 50 mer, the sensitivity for detecting a polymorphism in the PIK3CA gene is decreased; therefore, such a length is not preferred.
Further, the P5 and P5' fluorescently labeled oligonucleotides of the present invention preferably have a length of 10 mer to 40 mer, more preferably 12 mer to 30 mer, still more preferably 13 mer to 25 mer. By setting the length in the range of 10 mer to 40 mer, for example, the detection sensitivity tends to be increased.

The above-described P6 and P6' fluorescently labeled oligonucleotides of the present invention are required to have a length of 9 mer to 50 mer. When the P6 and P6' fluorescently labeled oligonucleotides have a length shorter than 9 mer or longer than 50 mer, the sensitivity for detecting a polymorphism in the PIK3CA gene is decreased; therefore, such a length is not preferred.
Further, the P6 and P6' fluorescently labeled oligonucleotides of the present invention preferably have a length of 9 mer to 40 mer, more preferably 11 mer to 30 mer, still more preferably 13 mer to 25 mer. By setting the length in the range of 9 mer to 40 mer, for example, the detection sensitivity tends to be increased.

The above-described P7 and P7' fluorescently labeled oligonucleotides of the present invention are required to have a length of 7 mer to 50 mer. When the P7 and P7' fluorescently labeled oligonucleotides have a length shorter than 7 mer or longer than 50 mer, the sensitivity for detecting a polymorphism in the PIK3CA gene is decreased; therefore, such a length is not preferred.
Further, the P7 and P7' fluorescently labeled oligonucleotides of the present invention preferably have a length of 7 mer to 40 mer, more preferably 10 mer to 30 mer, still more preferably 13 mer to 25 mer. By setting the length in the range of 7 mer to 40 mer, for example, the detection sensitivity tends to be increased.

The above-described P8 and P8' fluorescently labeled oligonucleotides of the present invention are required to have a length of 6 mer to 50 mer. When the P8 and P8' fluorescently labeled oligonucleotides have a length shorter than 6 mer or longer than 50 mer, the sensitivity for detecting a polymorphism in the PIK3CA gene is decreased; therefore, such a length is not preferred.
Further, the P8 and P8' fluorescently labeled oligonucleotides of the present invention preferably have a length of 7 mer to 40 mer, more preferably 10 mer to 30 mer, still more preferably 13 mer to 25 mer. By setting the length in the range of 7 mer to 40 mer, for example, the detection sensitivity tends to be increased.

The above-described P9 and P9' fluorescently labeled oligonucleotides of the present invention are required to have a length of 12 mer to 50 mer. When the P9 and P9' fluorescently labeled oligonucleotides have a length shorter than 12 mer or longer than 50 mer, the sensitivity for detecting a polymorphism in the PIK3CA gene is decreased; therefore, such a length is not preferred.
Further, the P9 and P9' fluorescently labeled oligonucleotides of the present invention preferably have a length of 12 mer to 40 mer, more preferably 12 mer to 30 mer, still more preferably 13 mer to 25 mer. By setting the length in the range of 12 mer to 40 mer, for example, the detection sensitivity tends to be increased.

The above-described P10 and P10' fluorescently labeled oligonucleotides of the present invention are required to have a length of 8 mer to 50 mer. When the P10 and P10' fluorescently labeled oligonucleotides have a length shorter than 8 mer or longer than 50 mer, the sensitivity for detecting a polymorphism in the PIK3CA gene is decreased; therefore, such a length is not preferred.
Further, the P10 and P10' fluorescently labeled oligonucleotides of the present invention preferably have a length of 8 mer to 40 mer, more preferably 11 mer to 30 mer, still more preferably 13 mer to 25 mer. By setting the length in the range of 8 mer to 40 mer, for example, the detection sensitivity tends to be increased.

The above-described P11 and P11' fluorescently labeled oligonucleotides of the present invention are required to have a length of 5 mer to 50 mer. When the P11 and P11' fluorescently labeled oligonucleotides have a length shorter than 5 mer or longer than 50 mer, the sensitivity for detecting a polymorphism in the PIK3CA gene is decreased; therefore, such a length is not preferred.
Further, the P1 1 and P11' fluorescently labeled oligonucleotides of the present invention preferably have a length of 7 mer to 40 mer, more preferably 10 mer to 30 mer, still more preferably 13 mer to 25 mer. By setting the length in the range of 7 mer to 40 mer, for example, the detection sensitivity tends to be increased.

By changing the base lengths of the P4 to P11' fluorescently labeled oligonucleotides, the Tm value, which is the dissociation temperature of a hybrid formed between the fluorescently labeled oligonucleotides and their respective target sequences, can be adjusted to a desired value.

In the present invention, the difference between the Tm value when each of the P1 to P11' fluorescently labeled oligonucleotides is hybridized with a wild-type sample nucleic acid and the Tm value when each of the P1 to P11' fluorescently labeled oligonucleotides is hybridized with a mutant-type sample nucleic acid is preferably not less than 1.5°C, more preferably not less than 2.0°C, still more preferably not less than 5.0°C, and yet still more preferably not less than 9.0°C.

Examples of a method of increasing the above-described difference in the Tm value include a method by which a probe is allowed to contain a base which mismatches with a base sequence corresponding to a region to which the probe hybridizes. Specific examples include those methods described in Nature Biotech (1997) vol. 15, pp. 331-335 and the like.

Table 3 shows examples of the base sequence of the above-described P4 to P11' fluorescently labeled oligonucleotides according to the present invention; however, the present invention is not restricted thereto.
Further, in Table 3, the underlined cytosines each represent the cytosine corresponding to the following base position of the base sequence indicated in SEQ ID NO:4 or 5: the 167th base for SEQ ID NO:11; the 164th base for SEQ ID NO:12; the 163rd base for SEQ ID NO:13; the 161st base for SEQ ID NOs: 14 and 15; the 160th base for SEQ ID NO:16; the 144th base for SEQ ID NO:17; the 148th base for SEQ ID NO:18; the 151st base for SEQ ID NO:19; the 144th and the 161st bases for SEQ ID NO:20; the 148th and the 164th bases for SEQ ID NO:21; and the 163rd base for SEQ ID NO:22.

**[Table 3]**

| | | | SEQ ID NO: |
|---|---|---|---|
| P4 | 5T-PIK3CA3140-Mt-R9 | (TAMRA)-cagccaccatgaCgtgc-P | 11 |
| P5 | 5T-PIK3CA3140-Mt-R10 | (TAMRA)-ccaccatgaCgtgcatca-P | 12 |
| P6 | 5T-PIK3CA3140-Mt-R11 | (TAMRA)-caccatgaCgtgcatca-P | 13 |
| P7 | ST-PIK3CA A3140G R1-17 | (TAMRA)-ccatgaCgtgcatcatt-P | 14 |
| P7 | 5PB-PIC3CA A3140G R1 | (Pacific Blue)-ccatgaCgtgcatcatt-P | 15 |
| P8 | 5T-PIK3CA3140-Mt-R12 | (TAMRA)-catgaCgtgcatcattcat-P | 16 |
| P9 | 3T-PIK3CA3140-Mt-R16 | accatgaCgtgcatcattc-(TAMRA) | 17 |
| P10 | 3T-PIK3CA3140-Mt-R17 | ccaccatgaCgtgcatc-(TAMRA) | 18 |
| P11 | 3T-PIK3CA3140-Mt-R18 | cagccaccatgaCgtgc-(TAMRA) | 19 |
| P7-P9 | 35T-PIK3CA-3140-R19 | (TAMRA)-ccatgaCgtgcatcattc-(TAMRA) | 20 |
| P5-P11 | 35T-PIK3CA-3140-R20 | (TAMRA)-ccaccatgaCgtgcatc-(TAMRA) | 21 |
| P6 | T-PIK3 CA-3140-R21 | c(c-(TAMRA))atgaCgtgcatcattc-P | 22 |

In the above-described P4 and P4' fluorescently labeled oligonucleotides of the present invention, it is required that the 167th base (cytosine) is labeled with a fluorescent dye.
In the above-described P5 and P5' fluorescently labeled oligonucleotides of the present invention, it is required that the 164th base (cytosine) is labeled with a fluorescent dye.
In the above-described P6 and P6' fluorescently labeled oligonucleotides of the present invention, it is required that the 163rd base (cytosine) is labeled with a fluorescent dye.
In the above-described P7 and P7' fluorescently labeled oligonucleotides of the present invention, it is required that the 161 st base (cytosine) is labeled with a fluorescent dye.
In the above-described P8 and P8' fluorescently labeled oligonucleotides of the present invention, it is required that the 160th base (cytosine) is labeled with a fluorescent dye.
In the above-described P9 and P9' fluorescently labeled oligonucleotides of the present invention, it is required that the 144th base (cytosine) is labeled with a fluorescent dye.
In the above-described P10 and P10' fluorescently labeled oligonucleotides of the present invention, it is required that the 148th base (cytosine) is labeled with a fluorescent dye.
In the above-described P11 and P1 1' fluorescently labeled oligonucleotides of the present invention, it is required that the 151 st base (cytosine) is labeled with a fluorescent dye.
The above-described P4 to P11' fluorescently labeled oligonucleotides may be used as a probe for detecting a polymorphism in the PIK3CA gene, particularly as a probe for detecting a polymorphism in the PIK3CA gene which detects an A3140G mutation.
In addition, the probe for detecting a polymorphism in the PIK3CA gene may be used as a probe for performing melting curve analysis.

More preferable embodiments of the P4 fluorescently labeled oligonucleotide and the P4' fluorescently labeled oligonucleotide include the following P4-1 fluorescently labeled oligonucleotide and the following P4'-1 fluorescently labeled oligonucleotide:
(P4-1) an oligonucleotide having an identity of at least 80% to a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye, the oligonucleotide recognizing a polymorphism at the 155th base of SEQ ID NO:4; and (P4'-1) an oligonucleotide which hybridizes under stringent conditions to the complementary strand of a base sequence complementary to a base sequence having a length of 13 to 50 bases including the 155th to the 167th bases of the base sequence indicated in SEQ ID NO:4 or 5, wherein the base corresponding to the 167th base is a cytosine labeled with a fluorescent dye, the oligonucleotide recognizing a polymorphism at the 155th base of SEQ ID NO:4.

More preferable embodiments of the P5 fluorescently labeled oligonucleotide and the P5' fluorescently labeled oligonucleotide include the following P5-1 fluorescently labeled oligonucleotide and the following P5'-1 fluorescently labeled oligonucleotide:
(P5-1) an oligonucleotide having an identity of at least 80% to a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye, the oligonucleotide recognizing a polymorphism at the 155th base of SEQ ID NO:4; and
(P5'-1) an oligonucleotide which hybridizes under stringent conditions to the complementary strand of a base sequence complementary to a base sequence having a length of 10 to 50 bases including the 155th to the 164th bases of the base sequence indicated in SEQ ID NO:4 or 5, wherein the base corresponding to the 164th base is a cytosine labeled with a fluorescent dye, the oligonucleotide recognizing a polymorphism at the 155th base of SEQ ID NO:4.

More preferable embodiments of the P6 fluorescently labeled oligonucleotide and the P6' fluorescently labeled oligonucleotide include the following P6-1 fluorescently labeled oligonucleotide and the following P6'-1 fluorescently labeled oligonucleotide:
(P6-1) an oligonucleotide having an identity of at least 80% to a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye, the oligonucleotide recognizing a polymorphism at the 155th base of SEQ ID NO:4; and
(P6'-1) an oligonucleotide which hybridizes under stringent conditions to the complementary strand of a base sequence complementary to a base sequence having a length of 9 to 50 bases including the 155th to the 163rd bases of the base sequence indicated in SEQ ID NO:4 or 5, wherein the base corresponding to the 163rd base is a cytosine labeled with a fluorescent dye, the oligonucleotide recognizing a polymorphism at the 155th base of SEQ ID NO:4.

More preferable embodiments of the P7 fluorescently labeled oligonucleotide and the P7' fluorescently labeled oligonucleotide include the following P7-1 fluorescently labeled oligonucleotide and the following P7'-1 fluorescently labeled oligonucleotide:
(P7-1) an oligonucleotide having an identity of at least 80% to a base sequence complementary to a base sequence having a length of 7 to 50 bases including the 155th to the 161st bases of the base sequence indicated in SEQ ID NO:4 or 5, wherein the base corresponding to the 161 st base is a cytosine labeled with a fluorescent dye, the oligonucleotide recognizing a polymorphism at the 155th base of SEQ ID NO:4; and
(P7'-1) an oligonucleotide which hybridizes under stringent conditions to the complementary strand of a base sequence complementary to a base sequence having a length of 7 to 50 bases including the 155th to the 161st bases of the base sequence indicated in SEQ ID NO:4 or 5, wherein the base corresponding to the 161st base is a cytosine labeled with a fluorescent dye, the oligonucleotide recognizing a polymorphism at the 155th base of SEQ ID NO:4.

More preferable embodiments of the P8 fluorescently labeled oligonucleotide and the P8' fluorescently labeled oligonucleotide include the following P8-1 fluorescently labeled oligonucleotide and the following P8'-1 fluorescently labeled oligonucleotide:
(P8-1) an oligonucleotide having an identity of at least 80% to a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye, the oligonucleotide recognizing a polymorphism at the 155th base of SEQ ID NO:4; and
(P8'-1) an oligonucleotide which hybridizes under stringent conditions to the complementary strand of a base sequence complementary to a base sequence having a length of 6 to 50 bases including the 155th to the 160th bases of the base sequence indicated in SEQ ID NO:4 or 5, wherein the base corresponding to the 160th base is a cytosine labeled with a fluorescent dye, the oligonucleotide recognizing a polymorphism at the 155th base of SEQ ID NO:4.

More preferable embodiments of the P9 fluorescently labeled oligonucleotide and the P9' fluorescently labeled oligonucleotide include the following P9-1 fluorescently labeled oligonucleotide and the following P9'-1 fluorescently labeled oligonucleotide:
(P9-1) an oligonucleotide having an identity of at least 80% to a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye, the oligonucleotide recognizing a polymorphism at the 155th base of SEQ ID NO:4; and
(P9'-1) an oligonucleotide which hybridizes under stringent conditions to the complementary strand of a base sequence complementary to a base sequence having a length of 12 to 50 bases including the 144th to the 155th bases of the base sequence indicated in SEQ ID NO:4 or 5, wherein the base corresponding to the 144th base is a cytosine labeled with a fluorescent dye, the oligonucleotide recognizing a polymorphism at the 155th base of SEQ ID NO:4.

More preferable embodiments of the P10 fluorescently labeled oligonucleotide and the P10' fluorescently labeled oligonucleotide include the following P10-1 fluorescently labeled oligonucleotide and the following P10'-1 fluorescently labeled oligonucleotide:
(P10-1) an oligonucleotide having an identity of at least 80% to a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye, the oligonucleotide recognizing a polymorphism at the 155th base of SEQ ID NO:4; and
(P10'-1) an oligonucleotide which hybridizes under stringent conditions to the complementary strand of a base sequence complementary to a base sequence having a length of 8 to 50 bases including the 148th to the 155th bases of the base sequence indicated in SEQ ID NO:4 or 5, wherein the base corresponding to the 148th base is a cytosine labeled with a fluorescent dye, the oligonucleotide recognizing a polymorphism at the 155th base of SEQ ID NO:4.

More preferable embodiments of the P11 fluorescently labeled oligonucleotide and the P11' fluorescently labeled oligonucleotide include the following P11-1 fluorescently labeled oligonucleotide and the following P11'-1 fluorescently labeled oligonucleotide:
(P11-1) an oligonucleotide having an identity of at least 80% to a base sequence complementary to a base sequence having a length of 5 to 50 bases including the 151st to the 155th bases of the base sequence indicated in SEQ ID NO:4 or 5, wherein the base corresponding to the 151 st base is a cytosine labeled with a fluorescent dye, the oligonucleotide recognizing a polymorphism at the 155th base of SEQ ID NO:4; and
(P11'-1) an oligonucleotide which hybridizes under stringent conditions to the complementary strand of a base sequence complementary to a base sequence having a length of 5 to 50 bases including the 151 st to the 155th bases of the base sequence indicated in SEQ ID NO:4 or 5, wherein the base corresponding to the 151st base is a cytosine labeled with a fluorescent dye, the oligonucleotide recognizing a polymorphism at the 155th base of SEQ ID NO:4.

<Primer>
In the below-described method of detecting a polymorphism in the PIK3 CA gene, primers are used in amplifying a sequence having a PIK3CA gene polymorphism to be detected by a PCR method.
The primers that may be used in the present invention are not particularly restricted as long as they are capable of amplifying a nucleic acid containing a base exhibiting at least one polymorphism selected from the group consisting of G1624A mutation, G1633A mutation and A3140G mutation, which are the target PIK3CA gene polymorphisms to be detected.

The primer to be applied to the PCR method is not particularly limited, as long as it is capable of amplifying a region to which the probe of the present invention may be hybridized. Such a primer may be designed based on the base sequences indicated in SEQ ID NO:1 to SEQ ID NO:5 by those skilled in the art. The length and Tm value of the primer may be a length from 12 mer to 40 mer and a value from 40°C to 70°C, or a length from 16 mer to 30 mer and a value from 55°C to 60°C.
The lengths of individual primers in a primer set do not need to be the same, although the Tm values of these primers are preferably approximately the same (or the difference between the Tm values of these primers is preferably within 5°C).

Examples of the primers that may be used for amplifying a base sequence containing a region to which the polymorphism detection probe according to the present invention used in the method of detecting a polymorphism according to the present invention hybridizes are shown below. It is noted here that the following examples are provided for illustrative purposes only and that, therefore, the present invention is not restricted thereto. In Table 4, Y represents C or T.

**[Table 4]**

| | Sequence (5'→3') | mer | SEQ ID NO: |
|---|---|---|---|
| PIK3CA ex9 F | gaacagctcaaagcaatttctacacgag | 28 | 23 |
| PIK3CA ex9 R | cagagaatYtccattttagcacttacYtgtgac | 33 | 24 |
| PIK3CA A3140G F | gaggctttggagtatttcatgaaacaaatg | 30 | 25 |
| PIK3CA A3140G R | gcatgctgtttaattgtgtggaagatccaatc | 32 | 26 |

When amplifying a base sequence containing a region to which the polymorphism detection probe according to the present invention hybridizes, from the standpoint of sensitivity and efficiency, for example, among the oligonucleotides shown in Table 4, the PIK3CA ex9 F and the PIK3CA ex9 R, or the PIK3CAA3140G F and the PIK3CAA3140G R may be used as a set of paired primers.

The method of detecting a polymorphism is not particularly limited, as long as it is a method in which a fluorescently labeled oligonucleotide as described above is used as a probe. As an example of the polymorphism detection method in which a fluorescently labeled oligonucleotide as described above is used as a probe, a method of detecting a polymorphism using Tm analysis is described below.

<Polymorphism Detection Method>
The method of detecting a polymorphism in the PIK3CA gene according to the present invention is a method of detecting a polymorphism in the PIK3CA gene which includes detecting a polymorphism in the PIK3CA gene by using at least one probe for detecting a polymorphism in the PIK3CA gene as described above.
The method of detecting a polymorphism of the present invention may include at least one probe for detecting a polymorphism as described above, and this may make it possible to detect a polymorphism(s) in the PIK3CA gene easily and with high sensitivity.
In addition, the method of detecting a polymorphism according to the present invention may be employed as a method of detecting a polymorphism in the PIK3CA gene, and may include the below-described processes or steps (I) to (IV), and may include the below-described step (V). The method of detecting a polymorphism according to the present invention has the feature of using the above-described probe, and other configurations, conditions and the like are not particularly limited by the description below.

Step (I): contacting the fluorescently-labeled probe with a single-stranded nucleic acid in a sample, to obtain a hybrid.
Step (II): dissociating the hybrid by changing the temperature of the sample containing the hybrid, and measuring the change in fluorescence signal due to the dissociation of the hybrid.
Step (III): measuring the Tm value, which is the dissociation temperature of the hybrid, based on the change in fluorescence signal.
Step (IV): detecting the presence of a polymorphism in the PIK3CA gene on the single-stranded nucleic acid in the sample, based on the Tm value.
Step (V): determining the abundance ratio of the single-stranded nucleic acid having the polymorphism in the total single-stranded nucleic acids contained in the sample, based on the presence of the polymorphism(s).

Furthermore, the method according to the present invention may further include amplifying the nucleic acid before obtaining the hybrid in step (I) or simultaneously with obtaining the hybrid in step (I), in addition to steps (I) to (IV) or in addition to steps (I) to (V).
The measurement of the Tm value in step (III) may include not only measuring the dissociation temperature of the hybrid, but also measuring the differential values of the fluorescence signal that changes according to the temperature when the hybrid is melted.

In the present invention, the nucleic acid in the sample may be a single-stranded nucleic acid or a double-stranded nucleic acid. In the case in which the nucleic acid is a double-stranded nucleic acid, the method may include, for example, melting (dissociating) the double-stranded nucleic acid in the sample into single-stranded nucleic acids by heating before being hybridized with the fluorescently-labeled probe. The dissociation of a double-stranded nucleic acid into single-stranded nucleic acids enables hybridization with the fluorescently-labeled probe.

In the present invention, the nucleic acid contained in the sample to be detected may be, for example, a nucleic acid originally contained in a biological sample, or an amplification product obtained by amplifying a region of the gene of interest that contains a mutated site(s) of the PIK3CA gene by PCR or the like using a nucleic acid originally contained in a biological sample as a template with a view to improving the detection accuracy. The length of the amplification product is not particularly limited, and may be, for example, a length from 50 mer to 1000 mer, or a length from 80 mer to 200 mer. Furthermore, the nucleic acid in the sample may be, for example, a cDNA that has been synthesized from RNAs derived from a biological sample (e.g., total RNAs, mRNAs, etc.) by RT-PCR (Reverse Transcription PCR).

In the present invention, the addition ratio (molar ratio) of the probe according to the present invention relative to the nucleic acids in the sample is not particularly limited. The amount of the probe to be added may be, for example, no more than 1-fold (by mol) of the amount of DNA in the sample. From the viewpoint of ensuring a sufficient detection signal, the addition ratio of the probe according to the present invention to be added relative to the nucleic acids in the sample (in a molar ratio) may be 0.1 or lower.
The "nucleic acids in the sample" may be, for example, the total nucleic acids to be detected that have the polymorphism to be detected and nucleic acids, other than the nucleic acids to be detected, that do not have the polymorphism, or the total amplification products containing a detection target sequence having the polymorphism to be detected and amplification products containing a sequence, other than the detection target sequence, that does not have the polymorphism. Although the ratio of the nucleic acid to be detected relative to nucleic acids in the sample is usually unknown in advance, the consequent addition ratio of the probe relative to the nucleic acids to be detected (or the amplification products containing a sequence to be detected) (in a molar ratio) may be 10 or lower. The addition ratio of the probe relative to the nucleic acids to be detected (or the amplification products containing a sequence to be detected) (in a molar ratio) may be 5 or lower, or 3 or lower. The lower limit of the ratio is not particularly limited, and may be, for example, 0.001 or higher, 0.01 or higher, or 0.1 or higher.

The above-described addition ratio of the fluorescently-labeled probe according to the present invention relative to the DNA may be, for example, a molar ratio relative to the double-stranded nucleic acids or a molar ratio relative to the single-stranded nucleic acids.

In the present invention, the measurement of the change in the signal caused by a temperature change for determining a Tm value may be carried out by measuring the absorbance at 260 nm on the basis of the principle described above. However, the measurement may be carried out by measuring a signal which is based on a signal from the label attached to the fluorescently-labeled probe, and which varies in accordance with the degree of formation of a hybrid of a single-stranded DNA and the probe. Therefore, the above-described fluorescently-labeled oligonucleotide may be used as the fluorescently-labeled probe. Examples of the fluorescently-labeled oligonucleotide (hereinafter sometimes collectively referred to as "fluorescently-labeled oligonucleotide") include a fluorescently-labeled oligonucleotide in respect of which the fluorescence intensity when the oligonucleotide is hybridized with a target sequence thereof is decreased (quenched) as compared to the fluorescence intensity when the oligonucleotide is not hybridized with the target sequence thereof, and a fluorescently-labeled oligonucleotide in respect of which the fluorescence intensity when the oligonucleotide is hybridized with a target sequence thereof is increased as compared to the fluorescence intensity when the oligonucleotide is not hybridized with the target sequence thereof.
The former fluorescently-labeled oligonucleotide does not show a fluorescence signal or only a weak fluorescence signal when the fluorescently-labeled oligonucleotide forms a hybrid (a double-stranded DNA) with the sequence to be detected; however, the fluorescently-labeled oligonucleotide shows a fluorescence signal or shows an increased fluorescence signal when the fluorescently-labeled oligonucleotide is dissociated by heating.
The latter fluorescently-labeled oligonucleotide shows a fluorescence signal when the fluorescently-labeled oligonucleotide forms a hybrid (a double-stranded DNA) with the sequence to be detected; however, the fluorescently-labeled oligonucleotide shows a decreased fluorescence signal or ceases to show a fluorescent signal when the fluorescently-labeled oligonucleotide is dissociated by heating. Therefore, similar to the measurement of the absorbance at 260 nm described above, the progress of melting can be monitored, and the Tm value can be determined by detecting the change in fluorescence signal from the fluorescent label under the conditions specific to the fluorescent label (for example, the fluorescence wavelength thereof).

The method for detecting the change in the signal based on a signal from the fluorescent dye in the polymorphism detection method according to the present invention is described below by way of specific examples. The polymorphism detection method according to the present invention has as a feature the use of the fluorescently-labeled polymorphism detection probe, and other processes and conditions of the method are not limited in any way.

The sample containing a nucleic acid that serves as a template for nucleic acid amplification is not particularly limited as long as the sample contains a nucleic acid, particularly the PIK3CA gene. Examples of such a sample include a sample that is derived from or can be derived from any biological source, examples of which include: a tissue such as colon or lung; a hemocyte such as a leukocyte cell; whole blood; plasma; sputum; a suspension of oral mucosa; a somatic cell of nail, hair or the like; a germ cell; milk; ascitic fluid; a paraffin-embedded tissue; gastric juice; gastric lavage fluid; urine; peritoneal fluid; amniotic fluid; and a cell culture. The method for sampling the sample, the method for preparing the sample containing a nucleic acid, and the like are not limited, and, conventional methods known in the art may be employed therefor. A nucleic acid obtained from such a biological source may be directly used as the template, or may be used after the sample has been subjected to pretreatment that modifies the properties of the sample.
For example, in the case in which whole blood is used as the sample, the isolation of genomic DNA from the whole blood may be carried out by a conventional method known in the art. For example, a commercially available genomic DNA isolation kit (trade name: GFX GENOMIC BLOOD DNA PURIFICATION KIT, available from GE Healthcare Biosciences), etc. may be used.

Next, a fluorescently-labeled polymorphism detection probe including the fluorescently-labeled oligonucleotide is added to the sample containing the isolated genomic DNA.
The ftuorescently-labeled probe may be added to a liquid sample containing the isolated genomic DNA, or may be mixed with the genomic DNA in an appropriate solvent. The solvent is not particularly limited, and examples of the solvent include conventional solvents known in the art, such as: a buffer solution such as Tris-HCl; a solvent containing at least one of KCl, MgCl₂, MgSO₄, or glycerol; and a PCR reaction solution.

The timing of the addition of the fluorescently-labeled probe is not particularly limited. For example, in a case in which an amplification process such as PCR described below is carried out, the ftuorescently-labeled probe may be added to the PCR amplification products after the amplification process is carried out, or may be added before the amplification process is carried out.
In the case in which the fluorescently-labeled probe is added before an amplification process such as PCR is carried out, for example, a fluorescent dye or a phosphate group may have been added to the 3' end of the probe, as described above.

The method of amplifying a nucleic acid may be, for example, a method in which a polymerase is employed. Examples thereof include a PCR method, an ICAN method, a LAMP method, and an NASBA method. In a case in which the amplification is carried out by a method in which a polymerase is employed, the amplification may be carried out in the presence of the fluorescently-labeled probe according to the present invention. Those skilled in the art would be able to easily adjust the reaction conditions of the amplification and the like in accordance with the fluorescently-labeled probe and the polymerase to be used. In the case in which the amplification is carried out in the presence of the fluorescently-labeled probe according to the present invention, a polymorphism can be detected by analyzing the Tm value of the ftuorescently-labeled probe only after the amplification of the nucleic acid is carried out, and, therefore, it is not necessary to separate the amplification product after completion of the reaction. Thus, contamination by (or of) the amplification product does not occur. In addition, since the detection can be carried out by the same apparatus as the apparatus required for the amplification, conveyance of a vessel is unnecessary, and automatization of the process is facilitated.

The DNA polymerase to be used in the PCR method may be selected, without particular limitation, from DNA polymerases that are usually used for PCR. Examples of the DNA polymerase include GENE TAQ (trade name, manufactured by NIPPON GENE CO., LTD.), PRIMESTAR MAX DNA POLYMERASE (trade name, manufactured by Takara Bio Inc.), and a Taq polymerase.
The amount of the polymerase to be used is not particularly limited as long as a usually-applied polymerase concentration is provided. For example, in the case in which a Taq polymerase is used, the concentration of the Taq polymerase may be, for example, a concentration from 0.01 U to 100 U relative to 50 µl of the reaction solution. In this range, for example, the sensitivity of the detection of the polymorphism in the PIK3CA gene tends to be increased.

The PCR method may be carried out under the conditions appropriately selected from usually-employed conditions.
When the amplification is carried out, the amplification may be monitored using real-time PCR so that the copy number of the DNA (a sequence to be detected) contained in the sample can be measured. In other words, the proportion of probes forming hybrids is increased as the amplification of the DNA (a sequence to be detected) by PCR proceeds, thereby changing the fluorescence intensity. By monitoring the change in fluorescence intensity, the copy number and/or the abundance ratio of the sequence to be detected (either a normal DNA or a mutant DNA) contained in the sample can be obtained.

In the polymorphism detection method according to the present invention, the fluorescently-labeled oligonucleotide and a single-stranded nucleic acid in the sample are brought into contact with each other, thereby allowing hybridization to occur. The single-stranded nucleic acid in the sample can be prepared by, for example, dissociating the PCR amplification products obtained in the above-described manner.

The heating temperature employed for dissociation of the PCR amplification products (the heating temperature in the dissociation process) is not particularly limited as long as it is a temperature at which the amplification products can be dissociated. For example, the heating temperature may be in the range from 85°C to 95°C. The heating time is not particularly limited, either. The heating time may be, for example, in the range from 1 second to 10 minutes, or from 1 second to 5 minutes.

The hybridization of the dissociated single-stranded DNA and the fluorescently-labeled oligonucleotide may be carried out by, for example, decreasing, after the dissociation process, the temperature from the heating temperature employed in the dissociation process. The temperature condition for the hybridization may be, for example, in the range from 40°C to 50°C.

The volume and concentration of each component in the reaction solution in the hybridization process are not particularly limited. In regard to specific examples thereof, the concentration of DNA in the reaction solution may be, for example, a concentration from 0.01 µM to 1 µM, or a concentration from 0.1 µM to 0.5 µM. The concentration of the fluorescently-labeled oligonucleotide may be, for example, in a range in which the above-described addition ratio relative to the DNA is satisfied, and may be, for example, a concentration from 0.001 µM to 10 µM, or a concentration from 0.001 µM to 1 µM.

The resultant hybrid of the single-stranded DNA and the fluorescently-labeled oligonucleotide is gradually heated, and the change in fluorescence signal caused by the temperature increase is measured. For example, in the case of using Q PROBE^{®}, the fluorescence intensity when the probe is hybridized with the single-stranded DNA is decreased (or quenched) as compared to the fluorescence intensity in the dissociated state. Therefore, for example, the hybrid emitting decreased fluorescence or the quenched hybrid may be gradually heated, and the increase in fluorescence intensity caused by the temperature increase may be measured.

The temperature range in which the change in fluorescence intensity is measured is not particularly limited, and the initial temperature may be, for example, a temperature from room temperature to 85°C, or a temperature from 25°C to 70°C. The final temperature may be, for example, a temperature from 40°C to 105°C. The rate of temperature increase is not particularly limited, either, and may be, for example, in the range from 0.1°C/sec to 20°C/sec, or in the range from 0.3°C/sec to 5°C/sec.

Next, the change in signal is analyzed to determine the Tm value. More specifically, the Tm value may be determined by calculating a differential value at each temperature (-d(Fluorescence Intensity)/dt) from the fluorescence intensity obtained, and taking the temperature at which the differential value has the lowest value as the Tm value. The Tm value may alternatively be determined as the point at which the increase in fluorescence intensity per unit time ((Increase in Fluorescence Intensity)/t) has the largest value. On the contrary, in the case in which a probe in relation to which the signal intensity is increased by the formation of the hybrid, rather than a quenching probe, is used as the fluorescently-labeled probe, the signal analysis and the determination of the Tm value may be carried out by measuring the decrease in fluorescence intensity.

In the present invention, the change in fluorescence signal caused by the temperature increase (preferably an increase in fluorescence intensity) may be measured while heating the hybrid as described above. However, instead of this method, the measurement of the change in signal may alternatively be carried out, for example, during the course of hybrid formation. In other words, the temperature of the sample, to which the probe has been added, may be decreased, and the change in fluorescence signal caused by the temperature decrease may be measured during the course of hybrid formation.

For example, in the case in which Q PROBE^{®} is used, the fluorescence intensity is high when the probe is added to the sample since the probe is in the dissociated state. However, when the hybrid is formed by a decrease in temperature, the fluorescence is decreased (or quenched). Therefore, for example, a decrease in fluorescence intensity caused by the temperature decrease may be measured while gradually decreasing the temperature of the heated sample.
On the other hand, in the case in which a probe in relation to which the signal therefrom is increased by hybrid formation is used, the fluorescence intensity is low (or quenched) when the probe is added to the sample since the probe is in the dissociated state. However, when the hybrid is formed by a decrease in temperature, the fluorescence intensity is increased. Therefore, for example, the increase in fluorescence intensity caused by the temperature decrease may be measured while gradually decreasing the temperature of the sample.

<Method of Evaluating Drug Efficacy>
The method of evaluating the efficacy of a drug according to the present invention includes detecting a polymorphism in the PIK3CA gene by the above-described polymorphism detection method; and evaluating the tolerance to an anticancer agent or the efficacy of an anticancer agent based on the results of the detection.
In the above-described method of detecting a polymorphism, a polymorphism in the PIK3CA gene is detected easily with high sensitivity by using the polymorphism detection probe according to the present invention; therefore, based on this polymorphism in the PIK3CA gene, an anticancer agent can be evaluated easily with high sensitivity.

In addition, the tolerance to an anticancer agent and its efficacy can be evaluated based on the presence or absence of a polymorphism and the abundance ratio of a mutant sequence and/or a normal sequence. Furthermore, the method of evaluating the efficacy of an anticancer agent according to the present invention is useful in determining whether a therapeutic strategy should be changed by, for example, increasing the dosage of the anticancer agent or using a different anticancer agent, based on the presence or absence of a mutation and the abundance ratio of a mutant sequence.
Here, specific examples of the anticancer agent to be evaluated include cetuximab and panitumumab.

<Reagent Kit for Detecting Polymorphism>
The reagent kit for detecting a polymorphism in the PIK3CA gene according to the present invention includes the above-described polymorphism detection probe(s).
This reagent kit for detecting a polymorphism includes at least one of the above-described polymorphism detection probes capable of easily detecting, in the PIK3CA gene, at least one polymorphism selected from the group consisting of G1624A mutation, G1633A mutation and A3140G mutation with high sensitivity; therefore, for example, the reagent kit is able to detect a polymorphism in the PIK3CA gene more easily.

In addition, the reagent kit for detecting a polymorphism according to the present invention may also include a primer for amplifying a base sequence containing the above-described PIK3CA gene polymorphism(s) to be detected. This enables the reagent kit for detecting a polymorphism according to the present invention to detect a polymorphism in the PIK3CA gene with good accuracy.
Here, with regard to the probe and primer that may be included in the reagent kit for detecting a polymorphism, those matters described above can be applied as they are.

In a case in which two or more types of fluorescently-labeled oligonucleotide are contained as the probes, the oligonucleotides may be contained as a mixture, or may be contained separately.
The two or more types of ftuorescently-labeled oligonucleotide may be respectively labeled with fluorescent dyes having different emission wavelengths from each other.
By using probes labeled with respectively different fluorescent dyes, detection of the signal from each ftuorescently-labeled oligonucleotide can simultaneously be carried out even in a single reaction system.

Besides the probe and the primers, the reagent kit according to the present invention may further include reagents required for carrying out nucleic acid amplification in the detection method according to the present invention. The probe, the primers and other reagents may be separately contained, or some of them may be contained as a mixture.
The term "separately contained" may refer to a state in which individual reagents are separated from each other such that the non-contact state therebetween is maintained, and does not necessarily require that the individual reagents be contained in separate containers that can be independently handled.
When the reagent kit includes a primer set for amplifying a base sequence including a base at the polymorphism site (a region to which the probe can hybridize), detection of the polymorphism with higher sensitivity, for example, can be achieved.

The reagent kit according to the present invention may further include an instruction manual that describes instructions for the generation of a differential melting curve for a sample containing a nucleic acid to be detected using the PIK3CA probe, and for the detection of a polymorphism in a gene-encoding base sequence through Tm value analysis based on the differential melting curve, or instructions that describes various reagents that are contained, or may additionally be contained, in the reagent kit.

### EXAMPLES

The present invention will now be described in detail by way of examples. However, the present invention is not limited to these examples in any way.

[Example 1]
Using a fully-automated SNP analyzer (trade name: I-DENSY (trademark); manufactured by ARKRAY, Inc.) and a test reagent having the formulation shown in Table 5 below, Tm analysis was performed to verify the performance of the respective polymorphism detection probes having the fluorescently labeled oligonucleotide shown in Table 6. Here, the type of fluorescent dye is indicated in parentheses for the respective probes. Further, in Table 6, capital letters indicate the bases representing the polymorphism. The same applies hereinafter.
As a template, the templates (oligo DNAs) shown in Table 7 were employed.

**[Table 5]**

| In 50 µL of reaction solution for Tm analysis: | |
|---|---|
| 1 × Gene Taq Universal Buffer (manufactured by NIPPON GENE CO., LTD.) | |
| 10 µM probe | 0.2 µM |
| 5 µM oligo DNA | 0.2 µM |

**[Table 6]**

| Forward probe | | | | | | |
|---|---|---|---|---|---|---|
| Name | Sequence (5'→3') | mer | Tm (Wt) | Tm (Mt) | 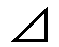 | SEQ ID NO: |
| 5T-PIK3CA3140-Wt-F3 | (TAMRA)-caaatgaatgatgcacAtca-P | 20 | 62 | 56 | 6.0 | 8 |
| 3T-PIK3CA3140-Wt-F8 | atgcacAtcatggtggc-(TAMRA) | 17 | 66 | 56.5 | 9.5 | 9 |
| T-PIK3CA-3140-F10 | atgcacAtcatggtgg(c-(TAMRA))t-P | 18 | 65 | 56 | 9.0 | 10 |

| Reverse probe | | | | | | |
|---|---|---|---|---|---|---|
| Name | Sequence (5'→3') | mer | Tm (Wt) | Tm (Mt) | 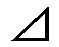 | SEQ ID NO: |
| 5T-PIK3CA3140-Mt-R9 | (TAMRA)-cagccaccatgaCgtgc-P | 17 | 57 | 70 | 13.0 | 11 |
| 5T-PIK3CA3140-Mt-R10 | (TAMRA)-ccaccatgaCgtgcatca-P | 18 | 55 | 67 | 12.0 | 12 |
| 5T-PIK3CA3140-Mt-R11 | (TAMRA)-caccatgaCgtgcatca-P | 17 | 52 | 64 | 12.0 | 13 |
| 5T-PIK3CA A3140G R1-17 | (TAMRA)-ccatgaCgtgcatcatt-P | 17 | 50 | 63 | 13.0 | 14 |
| 5T-PIK3CA3140-Mt-R12 | (TAMRA)-catgaCgtgcatcattcat-P | 19 | 54 | 64 | 10.0 | 16 |
| 3T-PIK3CA3140-Mt-R16 | accatgaCgtgcatcattc-(TAMRA) | 19 | 56 | 66 | 10.0 | 17 |
| 3T-PIK3CA3140-Mt-R17 | ccaccatgaCgtgcatc-(TAMRA) | 17 | 54 | 66 | 12.0 | 18 |
| 3T-PIK3CA3140-Mt-R18 | cagccaccatgaCgtgc-(TAMRA) | 17 | 58 | 70 | 12.0 | 19 |
| 35T-PIK3CA-3140-R19 | (TAMRA)-ccatgaCgtgcatcattc-(TAMRA) | 18 | 55 | 66.5 | 11.5 | 20 |
| 35T-PIK3CA-3140-R20 | (TAMRA)-ccaccatgaCgtgcatc-(TAMRA) | 17 | 55 | 68 | 13.0 | 21 |
| T-PIK3CA-3140-R21 | c(c-(TAMRA))atgaCgtgcatcattc-P | 18 | 52 | 64.5 | 12.5 | 22 |
| Tm values are actual measurements. | | | | | | |

**[Table 7]**

| Oligo DNA for forward probe (template) | | |
|---|---|---|
| Name | Sequence (5'→3') | SEQ ID NO: |
| PIK3CA A3140G Wt R (wild-type) | tccatttttgttgtccagccaccatgaTgtgcatcattcatttgtttcatgaaatactcc | 35 |
| PIK3CA A3140G Mt R (mutant-type) | tccatttttgttgtccagccaccatgaCgtgcatcattcatttgtttcatgaaatactcc | 36 |

| Oligo DNA for reverse probe (template) | | |
|---|---|---|
| Name | Sequence (5'→3') | SEQ ID NO: |
| PIK3CA A3140G Wt F (wild-type) | ggagtatttcatgaaacaaatgaatgatgcacAtcatggtggacaacaaaaatgga | 37 |
| PIK3CA A3140G Mt F (mutant-type) | ggagtatttcatgaaacaaatgaatgatgcacGtcatggtggacaacaaaaatgga | 38 |

The Tm analysis was performed by treating the reaction solution at 95°C for 1 second and then at 40°C for 60 seconds and subsequently measuring the change in the fluorescence intensity over time during a period in which the temperature of the solution was increased from 40°C to 80°C at a rate of 1°C/3 seconds. Since TAMRA was employed as a fluorescent dye, the excitation wavelength was in the range of 520 nm to 555 nm, and the detection wavelength was in the range of 585 nm to 700 nm. Based on these wavelengths, the changes in fluorescence intensity originating from the respective fluorescently labeled probes were measured.

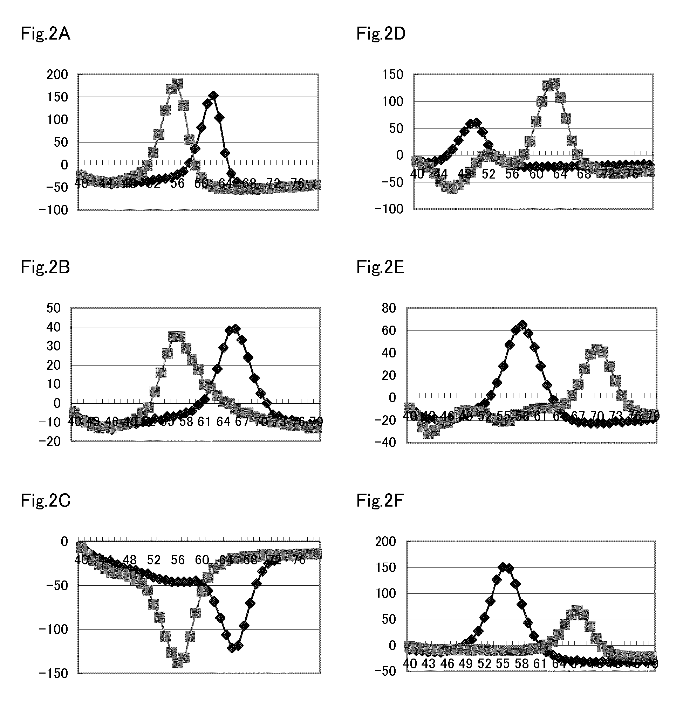

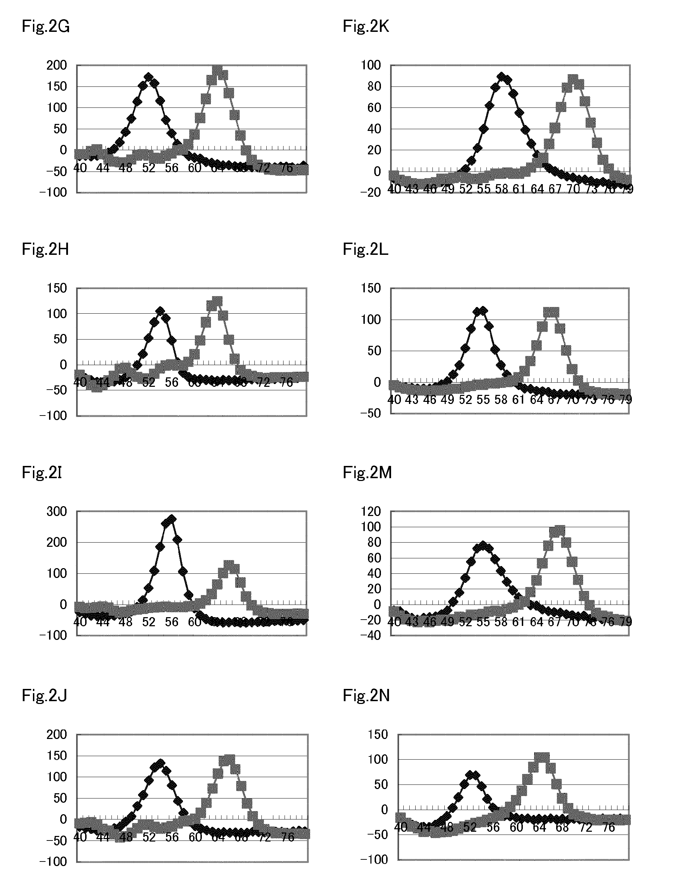

The Tm analysis yielded Fig. 2 showing the changes in the fluorescence value of the respective probes. In Fig. 2, the ordinate indicates the change in the fluorescence intensity per unit time (increase in the d-fluorescence intensity/t), and the abscissa indicates the temperature (°C). In Fig. 2, the patterns indicated with diamonds represent the results obtained by using, as a template, the artificial oligo sequence indicated in SEQ ID NO:35 having the same base sequence as indicated in SEQ ID NO:4 except that the 155th base (Y) is T and the results obtained by using the artificial oligo sequence indicated in SEQ ID NO:37 wherein the 155th base (Y) is A, which is complementary to T. Also, in Fig. 2, the patterns indicated with squares represent the results obtained using, as a template, the artificial oligo sequence indicated in SEQ ID NO:36 wherein the 155th base (Y) is C and the results obtained using the artificial oligo sequence indicated in SEQ ID NO:38 wherein the 155th base (Y) is G, which is complementary to C.

Further, in Fig. 2, (A) shows the results obtained using the probe according to the present invention indicated in SEQ ID NO:8; (B) shows the results obtained using the probe according to the present invention indicated in SEQ ID NO:9; (C) shows the results obtained using the probe according to the present invention indicated in SEQ ID NO:10; (D) shows the results obtained using the probe according to the present invention indicated in SEQ ID NO:14; (E) shows the results obtained using the probe according to the present invention indicated in SEQ ID NO:11; (F) shows the results obtained using the probe according to the present invention indicated in SEQ ID NO:12; (G) shows the results obtained using the probe according to the present invention indicated in SEQ ID NO:13; (H) shows the results obtained using the probe according to the present invention indicated in SEQ ID NO:16; (I) shows the results obtained using the probe according to the present invention indicated in SEQ ID NO:17; (J) shows the results obtained using the probe according to the present invention indicated in SEQ ID NO:18; (K) shows the results obtained using the probe according to the present invention indicated in SEQ ID NO:19; (L) shows the results obtained using the probe according to the present invention indicated in SEQ ID NO:20; (M) shows the results obtained using the probe according to the present invention indicated in SEQ ID NO:21; and (N) shows the results obtained using the probe according to the present invention indicated in SEQ ID NO:22.

From the results shown in Fig. 2, it was proven that, by using the fluorescently labeled oligonucleotide according to the present invention as a probe, a polymorphism at the 155th base of the base sequence indicated in SEQ ID NO:4 or 5 can be detected.

[Example 2]
Using the nucleic acid mixture shown in Table 8 (4 µl/reaction solution) or purified human genomic DNA (100 copies/reaction solution) as a sample and an automated SNP analyzer (trade name: I-DENSY (trademark); manufactured by ARKRAY, Inc.), PCR and Tm analysis were performed. The formulation of the PCR solution is shown in Table 9.

**[Table 8]**

| **Sample:** | | | |
|---|---|---|---|
| Number of copies of each plasmid in 4 µL of nucleic acid mixture: | | | |
| Plasmid name | Wt sample | Mt sample (1) | Mt sample (2) |
| exon9 Wt DNA | 1000 | 14000 | 18940 |
| G1624A mt DNA | 0 | 1000 | 60 |
| G1633AmtDNA | 0 | 5000 | 1000 |
| exon20 Wt DNA | 1000 | 9700 | 9970 |
| A3140G mt DNA | 0 | 300 | 30 |

**[Table 9]**

| **PCR solution** | |
|---|---|
| In 50 µL of PCR solution: | |
| 1 × PCR buffer | |
| Taq polymerase (in-house product) | 1.88 U |
| MgCl2 | 1.5 mmol/L |
| dNTP | 0.2 mmol/L |
| PIK3CA ex9 F | 0.5 µmol/L |
| PIK3CA ex9 R | 1 µmol/L |
| PIK3CA A3140G F | 2 µmol/L |
| PIK3CA A3140G R | 1 µmol/L |
| 5T-PIK3CA G1633A F1-19 | 0.1 µmol/L |
| 5FL-PIK3CA-G1624A-F1 | 0.1 µmol/L |
| 5PB-PIC3CA A3140G R1 | 0.1 µmol/L |

The PCR was performed by treating the reaction solution at 95°C for 60 seconds and then repeating 50 cycles of 95°C for 1 second and 58°C for 30 seconds.
Tm analysis was performed after the PCR by treating the reaction solution at 95°C for 1 second and then at 40°C for 60 seconds and subsequently measuring the change in fluorescence intensity over time during a period in which the temperature of the solution was increased from 40°C to 75°C at a rate of 1°C/3 seconds.

The polymorphism detection probes and primers are shown in Table 10.

**[Table 10]**

| Name | Sequence (5'→3') | mer | SEQ ID NO: |
|---|---|---|---|
| Primer | | | |
| PIK3CA ex9 F | gaacagctcaaagcaatttctacacgag | 28 | 23 |
| PIK3CA ex9 R | cagagaatYtccattttagcacttacYtgtgac | 33 | 24 |
| PIK3CA A3140G F | gaggctttggagtatttcatgaaacaaatg | 30 | 25 |
| PIK3CA A3140G R | gcatgctgtttaattgtgtggaagatccaatc | 32 | 26 |

| Probe | | | |
|---|---|---|---|
| 5T-PIK3CA G1633A F1-19 | (TAMRA)-ctctctGaaatcactAagc-P | 19 | 6 |
| 5FL-PIK3CA-G1624A-F1 | (BODIPY FL)-ctctctAaaatcactGagc-P | 19 | 7 |
| 5PB-PIC3CA A3140G R1 | (Pacific Blue)-ccatgaCgtgcatcatt-P | 17 | 14 |

| | | | |
|---|---|---|---|
| Y=C/T | | | |

The fluorescent dye, PACIFIC BLUE, has an excitation wavelength in the range of 365 nm to 415 nm and a detection wavelength in the range of 445 nm to 480 nm. The fluorescent dye, BODIPY FL, has an excitation wavelength in the range of 420 nm to 485 nm and a detection wavelength in the range of 520 nm to 555 nm. The fluorescent dye, TAMRA, has an excitation wavelength in the range of 520 nm to 555 nm and a detection wavelength in the range of 585 nm to 700 nm. Based on these wavelengths, the changes in fluorescence intensity originating from the respective fluorescently labeled probes were measured.

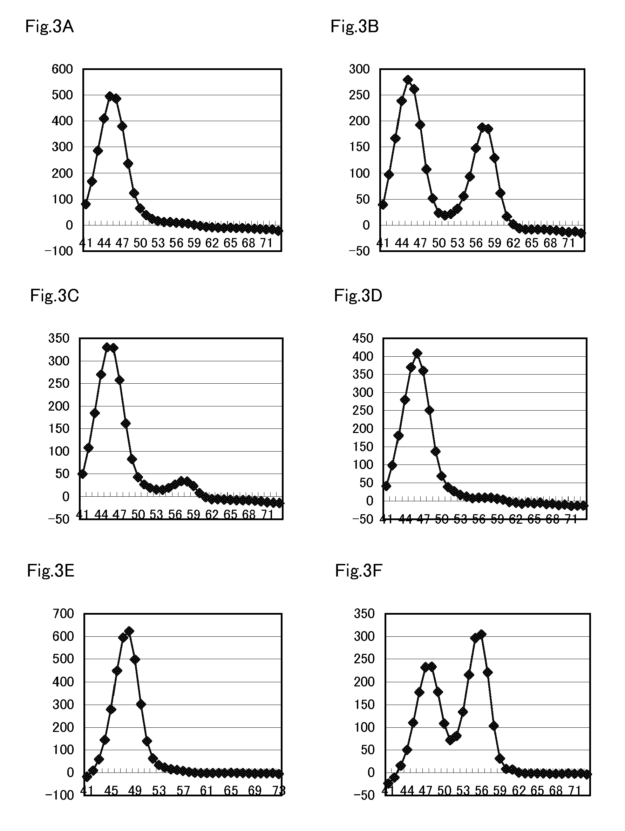

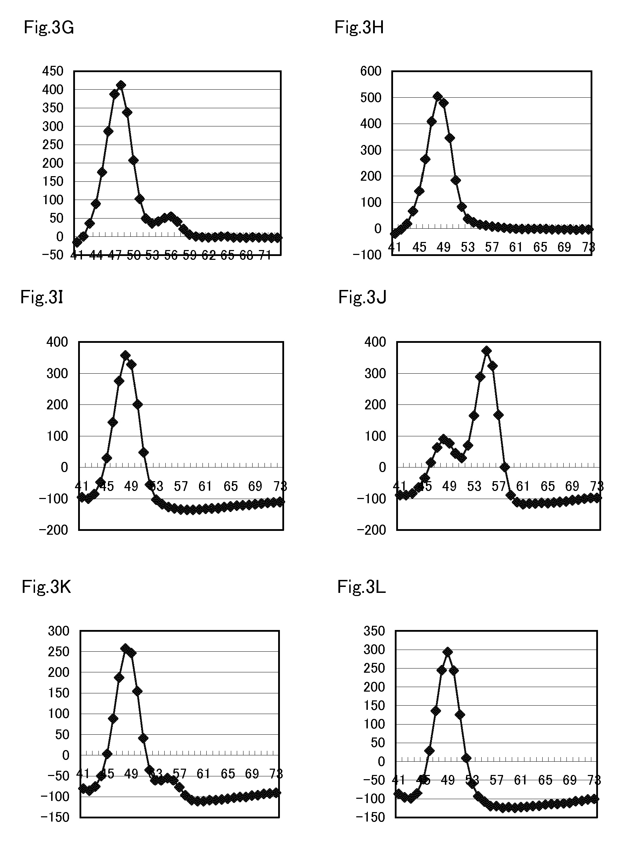

The Tm analysis yielded Fig. 3 showing the changes in the fluorescence value of the respective probes.
In Fig. 3, the ordinate indicates the change in fluorescence intensity per unit time (increase in d-fluorescence intensity/t), and the abscissa indicates the temperature (°C).

With regard to the A3140G mutation, one peak was observed at about 45°C for the wild-type sample (Wt sample); however, in the samples containing mutant-type plasmids (Mt sample (1) and Mt sample (2)), another peak was also observed at 57°C. With regard to the G1624A mutation and the G1633A mutation, one peak was observed at about 48°C for the wild-type sample (Wt sample); however, in the samples containing mutant-type plasmids (Mt sample (1) and Mt sample (2)), another peak was also observed at about 55°C.
From the above-described results, it was demonstrated that a polymorphism in the PIK3CA gene can be detected by using the probe according to the present invention.

[Comparative Example 1]
Using a fully-automated SNP analyzer (trade name: I-DENSY (trademark); manufactured by ARKRAY, Inc.) and a test reagent having the formulation shown in Table 11 below, Tm analysis was performed to verify the performance of the respective polymorphism detection probes having the fluorescently labeled oligonucleotide shown in Table 12. Here, the type of fluorescent dye is indicated in parentheses for the respective probes.
As a template, the templates (oligo DNAs) shown in Table 13 were employed.

**[Table 11]**

| In 50 µL of reaction solution for Tm analysis: | |
|---|---|
| 1 × Gene Taq Universal Buffer (manufactured by NIPPON GENE CO., LTD.) | |
| 10 µM probe | 0.2 µM |
| 5 µM oligo DNA | 0.2 µM |

**[Table 12]**

| Forward probe | | | | | | |
|---|---|---|---|---|---|---|
| Name | Sequence (5'→3') | mer | Tm (Wt) | Tm (Mt) | 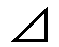 | SEQ ID NO: |
| 5T-PIK3CA A3140G F1-16 | (TAMRA)-cacGtcatggtggctg-P | 16 | 64 | 67 | 3 | 27 |
| 5T-PIK3CA3140-Wt-F5 | (TAMRA)-cAtcatggtggctggac-P | 17 | 65 | 61 | 4.0 | 28 |
| 3T-PIK3CA3140-Wt-F6 | cAtcatggtggctggacaac-(TAMRA) | 20 | 70 | 68 | 2.0 | 29 |
| 3T-PIK3CA3140-Wt-F7 | acAtcatggtggctggac-(TAMRA) | 18 | 68 | 64 | 4.0 | 30 |
| 3T-PIK3CA3140-Wt-F9 | caaatgaatgatgcacAtc-(TAMRA) | 19 | 61 | 59 | 2.0 | 31 |

| Reverse probe | | | | | | |
|---|---|---|---|---|---|---|
| Name | Sequence (5'→3') | mer | Tm (Wt) | Tm (Mt) | 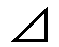 | SEQ ID NO: |
| 5T-PIK3CA3140-Mt-R7 | (TAMRA)-catttttgttgtccagccaccatgaCg-P | 27 | 69 | 73 | 4.0 | 32 |
| 5T-PIK3CA3140-Mt-R8 | (TAMRA)-ccagccaccatgaCgtg-P | 17 | 56 | 68.5 | 12.5 | 33 |
| 3T-PIK3CA3140-Mt-R15 | aCgtgcatcattcatttgtttc-(TAMRA) | 22 | 64 | 65 | 1.0 | 34 |

| | | | | | | |
|---|---|---|---|---|---|---|
| Tm values are actual measurements. | | | | | | |

**[Table 13]**

| Oligo DNA for forward probe (template) | | |
|---|---|---|
| Name | Sequence (5'→3') | SEQ ID NO: |
| PIK3CA A3140G Wt R (wild-type) | tccatttttgttgtccagccaccatgaTgtgcatcattcatttgtttcatgaaatactcc | 35 |
| PIK3CA A3140G Mt R (mutant-type) | tccatttttgttgtccagccaccatgaCgtgcatcattcatttgtttcatgaaatactcc | 36 |

| Oligo DNA for reverse probe (template) | | |
|---|---|---|
| Name | Sequence (5'→3') | SEQ ID NO: |
| PIK3CA A3140G Wt F (wild-type) | ggagtatttcatgaaacaaatgaatgatgcacAtcatggtggctggacaacaaaaatgga | 37 |
| PIK3CA A3140G Mt F (mutant-type) | ggagtatttcatgaaacaaatgaatgatgcacGtcatggtggctggacaacaaaaatgga | 38 |

The Tm analysis was performed by treating the reaction solution at 95°C for 1 second and then at 40°C for 60 seconds and subsequently measuring the change in fluorescence intensity over time during a period in which the temperature of the solution was increased from 40°C to 80°C at a rate of 1°C/3 seconds. Since TAMRA was employed as the fluorescent dye, the excitation wavelength was in the range of 520 nm to 555 nm, and the detection wavelength was in the range of 585 nm to 700 nm. Based on these wavelengths, the changes in fluorescence intensity originating from the respective fluorescently labeled probes were measured.

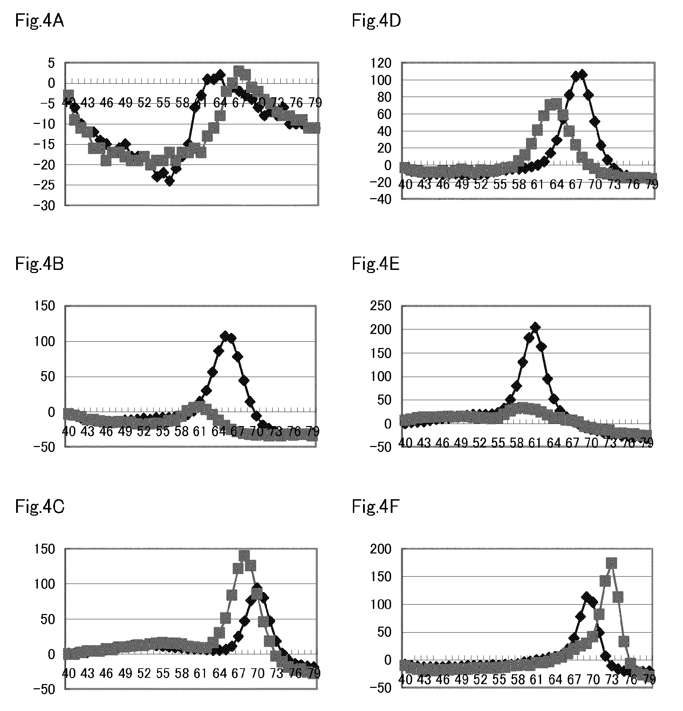

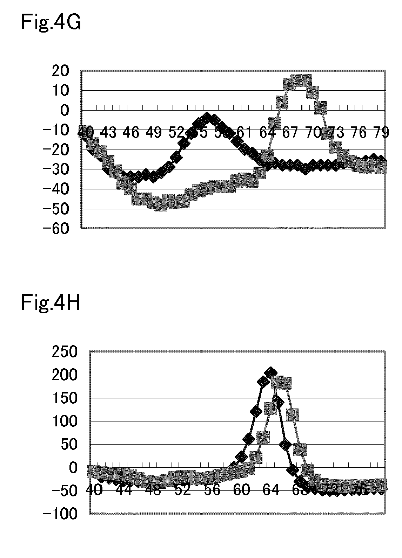

The Tm analysis yielded Fig. 4 showing the changes in the fluorescence value of the respective probes. In Fig. 4, the ordinate indicates the change in fluorescence intensity per unit time (increase in d-fluorescence intensity/t), and the abscissa indicates the temperature (°C).
In Fig. 4, the patterns indicated with diamonds represent the results obtained using, as a template, the artificial oligo sequence indicated in SEQ ID NO:35 having the same base sequence as indicated in SEQ ID NO:4 except that the 155th base (Y) is T and the results obtained using the artificial oligo sequence indicated in SEQ ID NO:37 wherein the 155th base (Y) is A, which is complementary to T.
Also, in Fig. 4, the patterns indicated with squares represent the results obtained using, as a template, the artificial oligo sequence indicated in SEQ ID NO:36 wherein the 155th base (Y) is C and the results obtained using the artificial oligo sequence indicated in SEQ ID NO:38 wherein the 155th base (Y) is G, which is complementary to C.

Further, in Fig. 4, (A) shows the results obtained using the probe indicated in SEQ ID NO:27; (B) shows the results obtained using the probe indicated in SEQ ID NO:28; (C) shows the results obtained using the probe indicated in SEQ ID NO:29; (D) shows the results obtained using the probe indicated in SEQ ID NO:30; (E) shows the results obtained using the probe indicated in SEQ ID NO:3 1; (F) shows the results obtained using the probe indicated in SEQ ID NO:32; (G) shows the results obtained using the probe indicated in SEQ ID NO:33; and (H) shows the results obtained using the probe indicated in SEQ ID NO:34.

From the results shown in Fig. 4, it was revealed that, in cases where the polymorphism detection probe according to the present invention was not used, a polymorphism in the PIK3CA gene could not be detected because, for example, at least one of the peaks was obscured or it was difficult to draw a distinction between the wild-type and the mutant-type due to the small temperature difference between the peaks. It is noted here that the fluorescently labeled oligonucleotides used in Comparative Example 1 were presented merely as representative examples of undetectable probes that exist in large numbers.

From the above-described results, it was demonstrated that, by the present invention, a polymorphism in the PIK3CA gene can be detected easily with high sensitivity.

## Claims

1. A probe for detecting a polymorphism in the PIK3CA gene, which is at least one fluorescently labeled oligonucleotide selected from the following P1 to P11':
(P1) an oligonucleotide having an identity of at least 80% to a base sequence having a length of 7 to 50 bases including the 226th to the 232nd bases of the base sequence indicated in any one of SEQ ID NOs:1 to 3, wherein the base corresponding to said 226th base is a cytosine labeled with a fluorescent dye;
(P1') an oligonucleotide which hybridizes under stringent conditions to the complementary strand of a base sequence having a length of 7 to 50 bases including the 226th to the 232nd bases of the base sequence indicated in any one of SEQ ID NOs:1 to 3, wherein the base corresponding to said 226th base is a cytosine labeled with a fluorescent dye;
(P2) an oligonucleotide having an identity of at least 80% to a base sequence having a length of 17 to 50 bases including the 139th to the 155th bases of the base sequence indicated in SEQ ID NO:4 or 5, wherein the base corresponding to said 139th base is a cytosine labeled with a fluorescent dye;
(P2') an oligonucleotide which hybridizes under stringent conditions to the complementary strand of a base sequence having a length of 17 to 50 bases including the 139th to the 155th bases of the base sequence indicated in SEQ ID NO:4 or 5, wherein the base corresponding to said 139th base is a cytosine labeled with a fluorescent dye;
(P3) an oligonucleotide having an identity of at least 80% to a base sequence having a length of 11 to 50 bases including the 155th to the 165th bases of the base sequence indicated in SEQ ID NO:4 or 5, wherein the base corresponding to said 165th base is a cytosine labeled with a fluorescent dye;
(P3') an oligonucleotide which hybridizes under stringent conditions to the complementary strand of a base sequence having a length of 11 to 50 bases including the 155th to the 165th bases of the base sequence indicated in SEQ ID NO:4 or 5, wherein the base corresponding to said 165th base is a cytosine labeled with a fluorescent dye;
(P4) an oligonucleotide having an identity of at least 80% to a base sequence complementary to a base sequence having a length of 13 to 50 bases including the 155th to the 167th bases of the base sequence indicated in SEQ ID NO:4 or 5, wherein the base corresponding to said 167th base is a cytosine labeled with a fluorescent dye;
(P4') an oligonucleotide which hybridizes under stringent conditions to the complementary strand of a base sequence that is itself complementary to a base sequence having a length of 13 to 50 bases including the 155th to the 167th bases of the base sequence indicated in SEQ ID NO:4 or 5, wherein the base corresponding to said 167th base is a cytosine labeled with a fluorescent dye;
(P5) an oligonucleotide having an identity of at least 80% to a base sequence complementary to a base sequence having a length of 10 to 50 bases including the 155th to the 164th bases of the base sequence indicated in SEQ ID NO:4 or 5, wherein the base corresponding to said 164th base is a cytosine labeled with a fluorescent dye;
(P5') an oligonucleotide which hybridizes under stringent conditions to the complementary strand of a base sequence that is itself complementary to a base sequence having a length of 10 to 50 bases including the 155th to the 164th bases of the base sequence indicated in SEQ ID NO:4 or 5, wherein the base corresponding to said 164th base is a cytosine labeled with a fluorescent dye;
(P6) an oligonucleotide having an identity of at least 80% to a base sequence complementary to a base sequence having a length of 9 to 50 bases including the 155th to the 163rd bases of the base sequence indicated in SEQ ID NO:4 or 5, wherein the base corresponding to said 163rd base is a cytosine labeled with a fluorescent dye;
(P6') an oligonucleotide which hybridizes under stringent conditions to the complementary strand of a base sequence that is itself complementary to a base sequence having a length of 9 to 50 bases including the 155th to the 163rd bases of the base sequence indicated in SEQ ID NO:4 or 5, wherein the base corresponding to said 163rd base is a cytosine labeled with a fluorescent dye;
(P7) an oligonucleotide having an identity of at least 80% to a base sequence complementary to a base sequence having a length of 7 to 50 bases including the 155th to the 161st bases of the base sequence indicated in SEQ ID NO:4 or 5, wherein the base corresponding to said 161 st base is a cytosine labeled with a fluorescent dye;
(P7') an oligonucleotide which hybridizes under stringent conditions to the complementary strand of a base sequence that is itself complementary to a base sequence having a length of 7 to 50 bases including the 155th to the 161st bases of the base sequence indicated in SEQ ID NO:4 or 5, wherein the base corresponding to said 161st base is a cytosine labeled with a fluorescent dye;
(P8) an oligonucleotide having an identity of at least 80% to a base sequence complementary to a base sequence having a length of 6 to 50 bases including the 155th to the 160th bases of the base sequence indicated in SEQ ID NO:4 or 5, wherein the base corresponding to said 160th base is a cytosine labeled with a fluorescent dye;
(P8') an oligonucleotide which hybridizes under stringent conditions to the complementary strand of a base sequence that is itself complementary to a base sequence having a length of 6 to 50 bases including the 155th to the 160th bases of the base sequence indicated in SEQ ID NO:4 or 5, wherein the base corresponding to said 160th base is a cytosine labeled with a fluorescent dye;
(P9) an oligonucleotide having an identity of at least 80% to a base sequence complementary to a base sequence having a length of 12 to 50 bases including the 144th to the 155th bases of the base sequence indicated in SEQ ID NO:4 or 5, wherein the base corresponding to said 144th base is a cytosine labeled with a fluorescent dye;
(P9') an oligonucleotide which hybridizes under stringent conditions to the complementary strand of a base sequence that is itself complementary to a base sequence having a length of 12 to 50 bases including the 144th to the 155th bases of the base sequence indicated in SEQ ID NO:4 or 5, wherein the base corresponding to said 144th base is a cytosine labeled with a fluorescent dye;
(P10) an oligonucleotide having an identity of at least 80% to a base sequence complementary to a base sequence having a length of 8 to 50 bases including the 148th to the 155th bases of the base sequence indicated in SEQ ID NO:4 or 5, wherein the base corresponding to said 148th base is a cytosine labeled with a fluorescent dye;
(P10') an oligonucleotide which hybridizes under stringent conditions to the complementary strand of a base sequence that is itself complementary to a base sequence having a length of 8 to 50 bases including the 148th to the 155th bases of the base sequence indicated in SEQ ID NO:4 or 5, wherein the base corresponding to said 148th base is a cytosine labeled with a fluorescent dye;
(P11) an oligonucleotide having an identity of at least 80% to a base sequence complementary to a base sequence having a length of 5 to 50 bases including the 151 st to the 155th bases of the base sequence indicated in SEQ ID NO:4 or 5, wherein the base corresponding to said 151 st base is a cytosine labeled with a fluorescent dye; or
(P11') an oligonucleotide which hybridizes under stringent conditions to the complementary strand of a base sequence that is itself complementary to a base sequence having a length of 5 to 50 bases including the 151 st to the 155th bases of the base sequence indicated in SEQ ID NO:4 or 5, wherein the base corresponding to said 151st base is a cytosine labeled with a fluorescent dye.

2. The probe according to claim 1, which is at least one fluorescently labeled oligonucleotide selected from the following P1-1 to P11'-1:
(P1-1) an oligonucleotide having an identity of at least 80% to a base sequence having a length of 7 to 50 bases including the 226th to the 232nd bases of the base sequence indicated in any one of SEQ ID NOs:1 to 3, wherein the base corresponding to said 226th base is a cytosine labeled with a fluorescent dye, said oligonucleotide recognizing a polymorphism of at least one base selected from the group consisting of the 232nd and the 241st bases of SEQ ID NO:1;
(P1'-1) an oligonucleotide which hybridizes under stringent conditions to the complementary strand of a base sequence having a length of 7 to 50 bases including the 226th to the 232nd bases of the base sequence indicated in any one of SEQ ID NOs:1 to 3, wherein the base corresponding to said 226th base is a cytosine labeled with a fluorescent dye, said oligonucleotide recognizing a polymorphism of at least one base selected from the group consisting of the 232nd and the 24 1 st bases of SEQ ID NO:1;
(P2-1) an oligonucleotide having an identity of at least 80% to a base sequence having a length of 17 to 50 bases including the 139th to the 155th bases of the base sequence indicated in SEQ ID NO:4 or 5, wherein the base corresponding to said 139th base is a cytosine labeled with a fluorescent dye, said oligonucleotide recognizing a polymorphism at the 155th base of SEQ ID NO:4;
(P2'-1) an oligonucleotide which hybridizes under stringent conditions to the complementary strand of a base sequence having a length of 17 to 50 bases including the 139th to the 155th bases of the base sequence indicated in SEQ ID NO:4 or 5, wherein the base corresponding to said 139th base is a cytosine labeled with a fluorescent dye, said oligonucleotide recognizing a polymorphism at the 155th base of SEQ ID NO:4;
(P3-1) an oligonucleotide having an identity of at least 80% to a base sequence having a length of 11 to 50 bases including the 155th to the 165th bases of the base sequence indicated in SEQ ID NO:4 or 5, wherein the base corresponding to said 165th base is a cytosine labeled with a fluorescent dye, said oligonucleotide recognizing a polymorphism at the 155th base of SEQ ID NO:4;
(P3'-1) an oligonucleotide which hybridizes under stringent conditions to the complementary strand of a base sequence having a length of 11 to 50 bases including the 155th to the 165th bases of the base sequence indicated in SEQ ID NO:4 or 5, wherein the base corresponding to said 165th base is a cytosine labeled with a fluorescent dye, said oligonucleotide recognizing a polymorphism at the 155th base of SEQ ID NO:4;
(P4-1) an oligonucleotide having an identity of at least 80% to a base sequence complementary to a base sequence having a length of 13 to 50 bases including the 155th to the 167th bases of the base sequence indicated in SEQ ID NO:4 or 5, wherein the base corresponding to said 167th base is a cytosine labeled with a fluorescent dye, said oligonucleotide recognizing a polymorphism at the 155th base of SEQ ID NO:4;
(P4'-1) an oligonucleotide which hybridizes under stringent conditions to the complementary strand of a base sequence that is itself complementary to a base sequence having a length of 13 to 50 bases including the 155th to the 167th bases of the base sequence indicated in SEQ ID NO:4 or 5, wherein the base corresponding to said 167th base is a cytosine labeled with a fluorescent dye, said oligonucleotide recognizing a polymorphism at the 155th base of SEQ ID NO:4;
(P5-1) an oligonucleotide having an identity of at least 80% to a base sequence complementary to a base sequence having a length of 10 to 50 bases including the 155th to the 164th bases of the base sequence indicated in SEQ ID NO:4 or 5, wherein the base corresponding to said 164th base is a cytosine labeled with a fluorescent dye, said oligonucleotide recognizing a polymorphism at the 155th base of SEQ ID NO:4;
(P5'-1) an oligonucleotide which hybridizes under stringent conditions to the complementary strand of a base sequence that is itself complementary to a base sequence having a length of 10 to 50 bases including the 155th to the 164th bases of the base sequence indicated in SEQ ID NO:4 or 5, wherein the base corresponding to said 164th base is a cytosine labeled with a fluorescent dye, said oligonucleotide recognizing a polymorphism at the 155th base of SEQ ID NO:4;
(P6-1) an oligonucleotide having an identity of at least 80% to a base sequence complementary to a base sequence having a length of 9 to 50 bases including the 155th to the 163rd bases of the base sequence indicated in SEQ ID NO:4 or 5, wherein the base corresponding to said 163rd base is a cytosine labeled with a fluorescent dye, said oligonucleotide recognizing a polymorphism at the 155th base of SEQ ID NO:4;
(P6'-1) an oligonucleotide which hybridizes under stringent conditions to the complementary strand of a base sequence that is itself complementary to a base sequence having a length of 9 to 50 bases including the 155th to the 163rd bases of the base sequence indicated in SEQ ID NO:4 or 5, wherein the base corresponding to said 163rd base is a cytosine labeled with a fluorescent dye, said oligonucleotide recognizing a polymorphism at the 155th base of SEQ ID NO:4;
(P7-1) an oligonucleotide having an identity of at least 80% to a base sequence complementary to a base sequence having a length of 7 to 50 bases including the 155th to the 161st bases of the base sequence indicated in SEQ ID NO:4 or 5, wherein the base corresponding to said 161 st base is a cytosine labeled with a fluorescent dye, said oligonucleotide recognizing a polymorphism at the 155th base of SEQ ID NO:4;
(P7'-1) an oligonucleotide which hybridizes under stringent conditions to the complementary strand of a base sequence that is itself complementary to a base sequence having a length of 7 to 50 bases including the 155th to the 161st bases of the base sequence indicated in SEQ ID NO:4 or 5, wherein the base corresponding to said 161st base is a cytosine labeled with a fluorescent dye, said oligonucleotide recognizing a polymorphism at the 155th base of SEQ ID NO:4;
(P8-1) an oligonucleotide having an identity of at least 80% to a base sequence complementary to a base sequence having a length of 6 to 50 bases including the 155th to the 160th bases of the base sequence indicated in SEQ ID NO:4 or 5, wherein the base corresponding to said 160th base is a cytosine labeled with a fluorescent dye, said oligonucleotide recognizing a polymorphism at the 155th base of SEQ ID NO:4;
(P8'-1) an oligonucleotide which hybridizes under stringent conditions to the complementary strand of a base sequence that is itself complementary to a base sequence having a length of 6 to 50 bases including the 155th to the 160th bases of the base sequence indicated in SEQ ID NO:4 or 5, wherein the base corresponding to said 160th base is a cytosine labeled with a fluorescent dye, said oligonucleotide recognizing a polymorphism at the 155th base of SEQ ID NO:4;
(P9-1) an oligonucleotide having an identity of at least 80% to a base sequence complementary to a base sequence having a length of 12 to 50 bases including the 144th to the 155th bases of the base sequence indicated in SEQ ID NO:4 or 5, wherein the base corresponding to said 144th base is a cytosine labeled with a fluorescent dye, said oligonucleotide recognizing a polymorphism at the 155th base of SEQ ID NO:4;
(P9'-1) an oligonucleotide which hybridizes under stringent conditions to the complementary strand of a base sequence that is itself complementary to a base sequence having a length of 12 to 50 bases including the 144th to the 155th bases of the base sequence indicated in SEQ ID NO:4 or 5, wherein the base corresponding to said 144th base is a cytosine labeled with a fluorescent dye, said oligonucleotide recognizing a polymorphism at the 155th base of SEQ ID NO:4;
(P10-1) an oligonucleotide having an identity of at least 80% to a base sequence complementary to a base sequence having a length of 8 to 50 bases including the 148th to the 155th bases of the base sequence indicated in SEQ ID NO:4 or 5, wherein the base corresponding to said 148th base is a cytosine labeled with a fluorescent dye, said oligonucleotide recognizing a polymorphism at the 155th base of SEQ ID NO:4;
(P10'-1) an oligonucleotide which hybridizes under stringent conditions to the complementary strand of a base sequence that is itself complementary to a base sequence having a length of 8 to 50 bases including the 148th to the 155th bases of the base sequence indicated in SEQ ID NO:4 or 5, wherein the base corresponding to said 148th base is a cytosine labeled with a fluorescent dye, said oligonucleotide recognizing a polymorphism at the 155th base of SEQ ID NO:4;
(P11-1) an oligonucleotide having an identity of at least 80% to a base sequence complementary to a base sequence having a length of 5 to 50 bases including the 151 st to the 155th bases of the base sequence indicated in SEQ ID NO:4 or 5, wherein the base corresponding to said 151 st base is a cytosine labeled with a fluorescent dye, said oligonucleotide recognizing a polymorphism at the 155th base of SEQ ID NO:4; or
(P11'-1) an oligonucleotide which hybridizes under stringent conditions to the complementary strand of a base sequence that is itself complementary to a base sequence having a length of 5 to 50 bases including the 151 st to the 155th bases of the base sequence indicated in SEQ ID NO:4 or 5, wherein the base corresponding to said 151st base is a cytosine labeled with a fluorescent dye, said oligonucleotide recognizing a polymorphism at the 155th base of SEQ ID NO:4.

3. The probe according to claim 1 or 2, wherein
said P1 or P1' fluorescently labeled oligonucleotide has the base corresponding to said 226th base labeled with a fluorescent dye at any one of the first to the third positions from the 5'-end;
said P2 or P2' fluorescently labeled oligonucleotide has the base corresponding to said 139th base labeled with a fluorescent dye at any one of the first to the third positions from the 5'-end;
said P3 or P3' fluorescently labeled oligonucleotide has the base corresponding to said 165th base labeled with a fluorescent dye at any one of the first to the third positions from the 3'-end;
said P4 or P4' fluorescently labeled oligonucleotide has the base corresponding to said 167th base labeled with a fluorescent dye at any one of the first to the third positions from the 5'-end;
said P5 or P5' fluorescently labeled oligonucleotide has the base corresponding to said 164th base labeled with a fluorescent dye at any one of the first to the third positions from the 5'-end;
said P6 or P6' fluorescently labeled oligonucleotide has the base corresponding to said 163rd base labeled with a fluorescent dye at any one of the first to the third positions from the 5'-end;
said P7 or P7' fluorescently labeled oligonucleotide has the base corresponding to said 161 st base labeled with a fluorescent dye at any one of the first to the third positions from the 5'-end;
said P8 or P8' fluorescently labeled oligonucleotide has the base corresponding to said 160th base labeled with a fluorescent dye at any one of the first to the third positions from the 5'-end;
said P9 or P9' fluorescently labeled oligonucleotide has the base corresponding to said 144th base labeled with a fluorescent dye at any one of the first to the third positions from the 3'-end;
said P10 or P10' fluorescently labeled oligonucleotide has the base corresponding to said 148th base labeled with a fluorescent dye at any one of the first to the third positions from the 3'-end; or
said P11 or P11' fluorescently labeled oligonucleotide has the base corresponding to said 151 st base labeled with a fluorescent dye at any one of the first to the third positions from the 3'-end.

4. The probe according to any one of claims 1 to 3, wherein
said P1 or Pa1' fluorescently labeled oligonucleotide has the base corresponding to said 226th base labeled with a fluorescent dye at the 5'-end;
said P2 or P2' fluorescently labeled oligonucleotide has the base corresponding to said 139th base labeled with a fluorescent dye at the 5'-end;
said P3 or P3' fluorescently labeled oligonucleotide has the base corresponding to said 165th base labeled with a fluorescent dye at the 3'-end;
said P4 or P4' fluorescently labeled oligonucleotide has the base corresponding to said 167th base labeled with a fluorescent dye at the 5'-end;
said P5 or P5' fluorescently labeled oligonucleotide has the base corresponding to said 164th base labeled with a fluorescent dye at the 5'-end;
said P6 or P6' fluorescently labeled oligonucleotide has the base corresponding to said 163rd base labeled with a fluorescent dye at the 5'-end;
said P7 or P7' fluorescently labeled oligonucleotide has the base corresponding to said 161st base labeled with a fluorescent dye at the 5'-end;
said P8 or P8' fluorescently labeled oligonucleotide has the base corresponding to said 160th base labeled with a fluorescent dye at the 5'-end;
said P9 or P9' fluorescently labeled oligonucleotide has the base corresponding to said 144th base labeled with a fluorescent dye at the 3'-end;
said P10 or P10' fluorescently labeled oligonucleotide has the base corresponding to said 148th base labeled with a fluorescent dye at the 3'-end; or
said P11 or P11' fluorescently labeled oligonucleotide has the base corresponding to said 151st base labeled with a fluorescent dye at the 3'-end.

5. The probe according to any one of claims 1 to 4, wherein said fluorescently labeled oligonucleotides emit fluorescence when not hybridized to a target sequence, and the fluorescence intensity of said fluorescently labeled oligonucleotides when hybridized to said target sequence is decreased or increased as compared to when not hybridized to said target sequence.

6. The probe according to any one of claims 1 to 5, which is a probe for melting curve analysis.

7. The probe according to any one of claims 1 to 6, which has at least one base sequence selected from the group consisting of SEQ ID NO:6, SEQ ID NO:7, SEQ ID NO:8, SEQ ID NO: 9, SEQ ID NO:10, SEQ ID NO:11, SEQ ID NO:12, SEQ ID NO:13, SEQ ID NO: 14, SEQ ID NO:15, SEQ ID NO: 16, SEQ ID NO: 17, SEQ ID NO: 18, SEQ ID NO: 19, SEQ ID NO:20, SEQ ID NO:21 and SEQ ID NO:22.

8. A method of detecting a polymorphism in the PIK3CA gene, which comprises the steps of:
(I) bringing the probe according to any one of claims 1 to 7 into contact with a single-stranded nucleic acid contained in a sample to allow hybridization of said fluorescently labeled oligonucleotide to said single-stranded nucleic acid, thereby obtaining a hybrid;
(II) dissociating said hybrid by changing the temperature of said sample containing said hybrid to measure the change in fluorescence signal caused by dissociation of said hybrid;
(III) determining the Tm value, which is the dissociation temperature of said hybrid, based on said change in fluorescence signal; and
(IV) based on said Tm value, detecting the presence of a polymorphism of the PIK3CA gene in said single-stranded nucleic acid in said sample.

9. The method according to claim 8, which further comprises the step of (V) based on said presence of a polymorphism, determining the abundance ratio of the single-stranded nucleic acid having said polymorphism in single-stranded nucleic acids contained in said sample.

10. The method according to claim 8 or 9, which further comprises the step of amplifying said nucleic acid prior to or simultaneously with said step (I).

11. A method of evaluating the efficacy of an anticancer agent, which comprises the steps of:
detecting a polymorphism in the PIK3CA gene by the method of detecting a polymorphism according to any one of claims 8 to 10; and
determining the efficacy of said anticancer agent based on the detected presence or absence of a polymorphism.

12. The method according to claim 11 wherein said anticancer agent is cetuximab or panitumumab.

13. A reagent kit for detecting a polymorphism in the PIK3CA gene, which comprises the probe according to any one of claims 1 to 7.

14. The reagent kit according to claim 13, which further comprises a primer for amplifying a base sequence containing a region to which the probe hybridizes.

15. Use of the reagent kit according to claim 13 or 14 in the detection of a polymorphism in the PIK3CA gene.
